(19) Europäisches Patentamt

European Patent Office

Office européen des brevets

(11) **EP 4 579 676 A1**

(12) **EUROPEAN PATENT APPLICATION**

(43) Date of publication:
**02.07.2025  Bulletin 2025/27**

(21) Application number: **24219474.4**

(22) Date of filing: **12.12.2024**

(51) International Patent Classification (IPC):
**G16H 20/40** (2018.01)  **G16H 40/63** (2018.01)
**G16H 50/20** (2018.01)  **G16H 50/70** (2018.01)

(52) Cooperative Patent Classification (CPC):
**G16H 20/40; G16H 40/63; G16H 50/20;
G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.12.2023  US 202363615836 P**

(71) Applicant: **Physio-Control, Inc.
Redmond, WA 98052 (US)**

(72) Inventors:
• **WALKER, Robert
Redmond, WA 98052 (US)**
• **SMITH, Robert
Redmond, WA 98052 (US)**

(74) Representative: **Schott, Jakob Valentin
Wuesthoff & Wuesthoff
Patentanwälte und Rechtsanwalt PartG mbB
Schweigerstraße 2
81541 München (DE)**

(54) **MEDICAL DEVICE WITH MODIFIABLE SONIFICATIONS**

(57)     A medical device configured to provide a real-time audio feedback for use during administration of an emergency medical treatment of a patient by medical personnel is disclosed. The medical device may comprise a memory, and a processor coupled to the memory. The processor may be configured to perform operations. The operations may include analyzing measurement data collected from the patient. The operations may also include associating a particular audio font based on a characteristic of the measurement data. The operations may further include identifying, based on the characteristic of the measurement data, one or more clinically meaningful thresholds in the measurement data. The operations may also include generating an audible change in the particular audio font to reflect whether a particular characteristic of the measurement data has crossed a clinically meaningful threshold of the one or more clinically-meaningful thresholds.

1400

1410 Analyzing, by a medical device, measurement data collected from a patient during administration of an emergency medical treatment of the patient by medical personnel

1420 Associating, by the medical device, a particular audio font based on a characteristic of the measurement data

1430 Identifying, by the medical device, based on the characteristic of the measurement data, one or more clinically meaningful thresholds in the measurement data

1440 Generating, by the medical device, an audible change in the particular audio font to reflect whether a particular characteristic of the measurement data has crossed a clinically meaningful threshold of the one or more clinically-meaningful thresholds

**FIG. 14**

## Description

### FIELD

The present disclosure relates generally to medical devices and, more particularly, to a medical device with modifiable sonifications.

### BACKGROUND

This background description is provided for the purpose of generally presenting the context of the disclosure. Unless otherwise indicated herein, material described in this section is neither expressly nor impliedly admitted to be prior art to the present disclosure or the appended claims.

Audio signals are used in a variety of ways to provide notifications, warnings, and so forth. For example, weather alerts on mobile devices, sirens indicating weather alerts, and/or natural disasters, police sirens, ambulance sirens, and so forth, are all used to indicate different types of warnings and/or notifications. Audio signals from medical devices also play a significant role in helping medical personnel.

Medical devices are used by medical personnel to monitor and treat patients, often in life-threatening emergency situations. For example, cardiac arrest or arrhythmia can be a life-threatening medical condition in which a person's heart fails to provide adequate blood flow to support life. During a cardiac arrest, the electrical activity of the heart may be disorganized (ventricular fibrillation (VF)), too rapid (ventricular tachycardia (VT)), absent (asystole), or organized at a normal or slow heart rate. A person in cardiac arrest may first lose their pulse, then consciousness, and finally the ability to breath. These events can happen in a short period of time. Changes in blood oxygen levels, blood sugar levels, blood pressure levels, are some other examples where timely intervention may be of utmost importance.

An automated external defibrillator (AED) is typically a small, portable device that analyzes the heart's rhythm and prompts a user to deliver medical treatment to the patient. Once the external defibrillator is activated, it can guide a user *(e.g.,* a first responder or rescuer) through each step of the treatment by providing audible and/or visual prompts that may include a cardiopulmonary resuscitation (CPR) treatment and/or a defibrillation treatment. The instructions provided by the external defibrillator to the user can improve the overall success rate of treating the person experiencing cardiac arrest.

Another type of external defibrillator is a larger portable device that incorporates a visual display presenting an electrocardiogram (ECG) waveform allowing a trained medical provider to analyze the patient's heart rhythm themselves, and make their own decisions regarding delivery of treatments and timing of those treatments. Such "manual" external defibrillators often incorporate additional physiologic monitoring parameters, allowing them to be useful in assessing and treating a much wider variety of patients than just cardiac arrest patients. Such defibrillators are often referred to as "multi-parameter monitor/defibrillators". The additional monitoring parameters may include, but are not limited to: additional ECG "leads" (i.e. the cardiac electrical signal as measured from different combinations of ECG electrodes in different locations on the patient's body, providing different perspectives on the cardiac electrical activity), pulse oximetry (which non-invasively measures a blood oxygen saturation, reported as an SpO2 value, typically with an accompanying waveform display that is reflective of the quality of the physiologic signal being processed to generate the SpO2 value), capnography (which measures the concentration or partial pressure of $CO_2$ in the exhaled gas of the patient's breath, reported as an end-tidal CO2 value - EtCO2 - typically with an accompanying waveform display that is reflective of the continuously changing level of $CO_2$ in the patient's airway), and blood pressure, as measured either non-invasively (*e.g.* via an oscillometric technique) or invasively. Additional potential physiologic monitoring parameters will be evident to those skilled in the art of emergency medical monitoring.

Multi-parameter monitor/defibrillators are used to assess and treat a wide variety of types of patients. For example, in some patients who are not in cardiac arrest, there is an emergent need to provide advanced airway management that may include performing endotracheal intubation, often with the assistance of medications that render the patient unconscious and unable to breath on their own. Such procedures can be particularly challenging, especially in an emergency medical setting, due to the large number of discrete steps and decisions that need to be made within a short period of time. This heighted cognitive load on the care provider can have numerous consequences on the safety and efficacy of the procedure. One common example of such adverse consequences is decreased or absent provider awareness of important changes in the physiologic status of the patient - for example, a decreasing oxygen saturation (SpO2) that has reached a critical threshold of clinical significance, or a change in the characteristics of the CO2 waveform indicating that the placement, seal, and/or another attribute of the airway has been partially, but not necessarily completely compromised, potentially resulting in compromised oxygenation and ventilation of the patient.

In medical care scenarios such as those described above, sound is sometimes used to communicate information to the care provider. For example, audible alarms are a common feature on physiologic monitors.

To illustrate further in the context of an oxygen saturation (SpO2) monitor, sounding tones corresponding to photoplethysmographic peaks can be used to convey information about the patient's pulse rate. SpO2 values indicate an amount of oxygen in the blood as a percentage of a maximum capacity in the blood. SpO2 values may be conveyed by changing the tone pitch with respect to those SpO2 values displayed on the monitor. Audible monitoring enhances patient care by conveying useful information without demanding attention to the device display. In order to be useful, clear discrimination of the audible information is needed. Generally, tone pitches may be substantially similar in frequency, thereby resulting in an inability to differentiate between SpO2 values. Some conventional devices require an SpO2 change of 6% for successful audible discrimination by users. The necessity of such a large change in the SpO2 value for a typical user to begin to be able to perceive the changing physiologic status of the patient can be particularly problematic in some situations, such as ones (*e.g.* medication-facilitated endotracheal intubation, as described above) where the patient's physiologic status is known to commonly deteriorate (*e.g.* oxygen desaturation), and earlier awareness of the rate and progression of such deterioration can allow earlier response to and correction of such deterioration, avoiding negative sequelae that might otherwise occur.

For example, pulse oximeters that provide pulse tones of variable pitch dependent on the measured oxygen saturation typically use a linear mapping between oxygen saturation and the sonic frequency of the pulse tone. In this scheme, the entire 100% to 0% range of possible oxygen saturation values is mapped across a range of approximately 2 musical octaves. Because human psychoacoustic perception of the relationship between the frequency of a sound and its musical pitch is logarithmic, rather than linear, the aforementioned linear mapping scheme produces dramatically different behaviors at opposite ends of the range of possible oxygen saturation values. The change from 100% saturation to 90% saturation is mapped to a pitch change of less than a half tone, while the change from 30% saturation to 0% saturation is allocated nearly the entire lower octave. This is a more than 11-fold difference that furthermore arguably emphasizes an incorrect (both less clinically relevant and less accurately measured) end of the range of possible saturation values.

While the linear mapping of saturation to sonic frequency may be reasonable in many monitoring situations, it can be suboptimal and even potentially hazardous during certain emergency care procedures. For example, patients undergoing rapid sequence intubation (RSI) are at high risk for precipitous oxygen desaturation that need to be promptly detected and corrected. The alternative can result in significant patient harm, including cardiac arrest. For an RSI patient with an initial oxygen saturation near 100%, a decrease in finger pulse oximeter oxygen saturation to 90% van be an indication that the arterial saturation is already likely well below 90%, and in a danger zone. In this circumstance, using a pitch change of less than a half tone to indicate the difference between "perfect" and "extreme danger" - especially when the intubator is cognitively distracted by the act of intubation - can make a difference between life and death for a patient.

Although changes in pitch and intensity are used, in many situations - especially in life-threatening, time sensitive situations - such traditional audible sound variations fail to convey the urgency of the situation. For example, the changes in pitch and intensity may be minimal or indistinguishable, causing a miss and/or a delay in providing appropriate medical attention, thereby losing a critical window of opportunity during a patient's care delivery. Also, for example, the few seconds involved in viewing the measurement data on the display of the medical device may make a difference between life and death. As another example, having to review the displayed measurement data to determine reliability or unreliability of a particular measurement may take away precious time that can be better spent in delivering care.

## SUMMARY

The present application is directed to embodiments that relate to systems, methods, and apparatus to provide a real-time audio feedback for use during administration of an emergency medical treatment of a patient by medical personnel. Some medical devices (*e.g.*, pulse oximeters) have traditionally provided an audible beep that is proportional to the pulse and SpO2 value. An audible signal may be configured to vary in pitch depending on the SpO2 values. However, a manual review of the text and digital data is generally required to confirm specific values and determine whether the values have passed a specific threshold.

As described in some examples herein, an alternate mapping of oxygen saturation values to pulse tone pitch can be more cognizant of the clinical significance of different oxygen saturation values during critical procedures like RSI, and more aligned with the way that humans perceive different sonic frequencies. Such an alternate mapping may be used in all monitoring situations, or may be selectively invoked when pertinent critical emergency procedures such as RSI are being performed. Such selective invocation may be automatically tied to use of a specific monitoring mode (*e.g.*, an "intubation assist mode"), or use of a specific tool (*e.g.*, a connected video laryngoscope).

In some embodiments, the existing two octaves of potential pitches may be mapped to the 100% to 0% range of potential oxygen saturation values linearly in terms of musical interval, rather than linearly in terms of sonic frequency. This can facilitate greater perception of oxygen saturation changes at the higher end of the saturation range, where most patients are going to be operating, and where it is impactful for situational awareness to prevent or rapidly respond to dangerous desaturation during critical procedures like RSI. In a slightly modified embodiment, every round increment change in oxygen saturation (say, every 5%) may be mapped to a fixed number of precise musical pitch steps (say, exactly one musical whole tone per 5% change in saturation), so that a user with good relative pitch perception may easily perceive a quantitative change in oxygen saturation, and users with perfect pitch may be able to perceive an absolute oxygen saturation without having to look at the monitor to read the measurement values. The magnitude of pitch change per unit change in oxygen saturation could be even further exaggerated at the high end of the oxygen saturation range - for example, one musical whole tone per 1% or 2% change in saturation - since it is of much greater clinical importance to recognize incipient and early-stage desaturation, than to be able to perceive the fine detail of SpO2 changes that are already deep in the "danger zone" of oxygen saturation values.

In some embodiments, the audio fonts may be used as a continuously changing parameter, rather than just one that makes a step change at one or more critical thresholds. For example, similar to mapping pitch to SpO2 as a continuous function, the audio font may be mapped to signal quality (or some other signal characteristic) in a continuously varying way. In some embodiments, a multiple critical threshold scheme may be viewed as a discrete example of such a "continuously-varying" case, since multiple thresholds are "coarsely varying via multiple discrete steps."

In some embodiments, a medical device can be configured to analyze collected patient data and generate audio signals indicative of a state of a patient's medical condition and/or a reliability of the analyzed data. As a non-limiting example, the medical device (*e.g.,* a defibrillator, a patient monitoring device, a monitordefibrillator such as the LIFEPAK 35 (LP35), a blood pressure monitor, an SpO2 device, etc.) can be configured to collect patient data of a patient and transmit audio or visual signals that can allow medical personnel to become aware of a patient's critical health status (*e.g.,* in the midst of a stressful/chaotic patient care event) without having to review text or digital data displayed on the medical device.

Signal quality of the waveform representing measurement data may be another issue that can be communicated. Especially in emergency care situations, SpO2 measurements calculated by a pulse oximeter may sometimes be erroneous, and many pulse oximeters can hold a stale value for longer than what may be considered safe. For example, a falsely reassuring high number may be displayed by the medical device. Caregivers have to review the displayed waveform to determine a quality of the waveform, before they can accept or reject the displayed value. A distinct audio font that indicates signal quality can remedy this situation, and provide a real-time audible notification of a quality of the displayed waveform.

Accordingly, as described herein, a medical device, such as a defibrillator can be configured to add an additional dimension of audio feedback, via "audio fonts" corresponding to threshold SpO2 values. The term "audio fonts" as used herein, generally refers to distinct overtones or timbral signatures (*e.g.*, harmonic, polyphonic, or monophonic timbres) that can be applied to the audio feedback. Such audio tones may differentiate between two sounds when they are in the same frequency. Such audio fonts can be applied at clinically-meaningful measurement thresholds (*e.g.*, SpO2 value thresholds) to more clearly indicate to a user that measurement values (*e.g.,* SpO2 values) are not only changing, but have crossed a critical threshold. Also, for example, the audio fonts can be applied to indicate whether a reliability of measurement data has changed. Such an audio font, especially during an emergency episode, may alert a user or rescuer to perform appropriate cardiac resuscitation, or airway management treatments, such as CPR treatments, re-oxygenation treatments, or airway placement confirmation/adjustment, on a person or patient. Awareness that the values have crossed a critical threshold may alter real-time care and treatment decisions by medical personnel. The pitch, intensity and the audio font of the SpO2 tones represent different sonifications of physiologic and device information.

Although the examples described are in the context of medical devices, this is for illustrative purposes only. The concept of audio fonts described herein may be applicable to any type of audio signal, alert, and/or notification. For example, audio fonts may be applied to weather alerts on mobile devices, sirens indicating weather alerts, and/or natural disasters, police sirens, ambulance sirens, and so forth, to indicate varying levels of severity, to prompt different responses, and so forth.

Another application of the audio fonts may be to indicate signal quality for incoming measurement data. Signal quality of a waveform representing measurement data may be an issue that can be communicated. Especially in emergency care situations, SpO2 measurements calculated by a pulse oximeter may sometimes be erroneous, and some pulse oximeters may hold a stale value for longer than what may be considered safe. For example, a falsely reassuring high number may be displayed by the medical device. Caregivers may have to review the displayed waveform to determine a quality of the waveform, before they can accept or reject the displayed value. A distinct audio font that indicates signal quality can remedy this situation, and provide a real-time audible notification of a quality of the displayed waveform.

In one aspect, a medical device configured to provide a real-time audio feedback for use during administration of an emergency medical treatment of a patient by medical personnel is disclosed. The medical device may comprise a memory, and a processor coupled to the memory. The processor may be configured to perform operations. The operations may include analyzing measurement data collected from the patient. The operations may also include associating a particular audio font based on a characteristic of the measurement data. The operations may further include identifying, based on the characteristic of the measurement data, one or more clinically meaningful thresholds in the measurement data. The operations may also include generating an audible change in the particular audio font to reflect whether a particular characteristic of the measurement data has crossed a clinically meaningful threshold of the one or more clinically-meaningful thresholds.

In another aspect, a method to provide a real-time audio feedback for use during administration of an emergency medical treatment of a patient by medical personnel is disclosed. The method may involve analyzing, by a medical device, measurement data collected from the patient. The method may also involve associating, by the medical device, a particular audio font based on a characteristic of the measurement data. The method may additionally involve identifying, by the medical device, based on the characteristic of the measurement data, one or more clinically meaningful thresholds in the measurement data. The method may also involve generating, by the medical device, an audible change in the particular audio font to reflect whether a particular characteristic of the measurement data has crossed a clinically meaningful threshold of the one or more clinically-meaningful thresholds.

In still another aspect, a non-transitory computer-readable medium configured to provide a real-time audio feedback for use during administration of an emergency medical treatment of a patient by medical personnel is disclosed. The non-transitory computer-readable medium may include a memory and a processor coupled to the memory. The processor may be configured to perform operations. The operations may include analyzing, by a medical device, measurement data collected from the patient. The operations may also include associating, by the medical device, a particular audio font based on a characteristic of the measurement data. The operations may further include identifying, by the medical device and based on the characteristic of the measurement data, one or more clinically meaningful thresholds in the measurement data. The operations may also include generating, by the medical device, an audible change in the particular audio font to reflect whether a particular characteristic of the measurement data has crossed a clinically meaningful threshold of the one or more clinically-meaningful thresholds.

The foregoing summary is illustrative only and is not intended to be in any way limiting. In addition to the illustrative aspects, embodiments, and features described above, further aspects, embodiments, and features will become apparent by reference to the figures and the following detailed description.

## BRIEF DESCRIPTION OF THE DRAWINGS

A more complete understanding of embodiments of the present application may be derived by referring to the detailed description and claims when considered in conjunction with the following figures, wherein like reference numbers may refer to similar elements throughout the figures. The figures are provided to facilitate understanding of the disclosure without limiting the breadth, scope, scale, or applicability of the disclosure. The drawings are not necessarily made to scale.

FIG. 1 is a diagram illustrating a representation of an example scene showing the use of a medical device for monitoring and delivering treatment to a person or patient experiencing a medical condition, in accordance with an example implementation;

FIG. 2 illustrates a front view of a medical device, in accordance with an example implementation;

FIG. 3 illustrates a simplified block diagram of the components of a medical device, in accordance with an example implementation;

FIG. 4 illustrates a graphical user interface of a medical device, in accordance with an example implementation;

FIG. 5 is an example graph illustrating SpO2 values, in accordance with an example implementation;

FIG. 6 is a comparison of a scaled graph and a linear graph for SpO2 values, in accordance with an example implementation;

FIG. 7 illustrates an example hybrid pitch scale for SpO2 values, in accordance with an example implementation;

FIG. 8 illustrates an example dissociation curve for multiple ranges of SpO2 values, in accordance with an example implementation;

FIG. 9A illustrates an example table for a first set of pitch values for multiple ranges of SpO2 values, in accordance with an example implementation;

FIG. 9B illustrates an example table for a second set of pitch values, in accordance with an example implementation;

FIG. 10 illustrates an example interface to generate a modified sonification for a particular SpO2 value in a first range, in accordance with an example implementation;

FIG. 11 illustrates an example interface to generate a modified sonification for a particular SpO2 value in a second range, in accordance with an example implementation;

FIG. 12 illustrates an example interface to generate a modified sonification for a particular SpO2 value in a third range, in accordance with an example implementation; and

FIG. 13 illustrates training and inference phases for a machine learning model, in accordance with an example implementation.

FIG. 14 is a flowchart illustrating a method, in accordance with an example implementation.

EP 4 579 676 A1

## DETAILED DESCRIPTION

The figures and the following description illustrate specific exemplary embodiments. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific embodiments or examples described below, but by the claims and their equivalents.

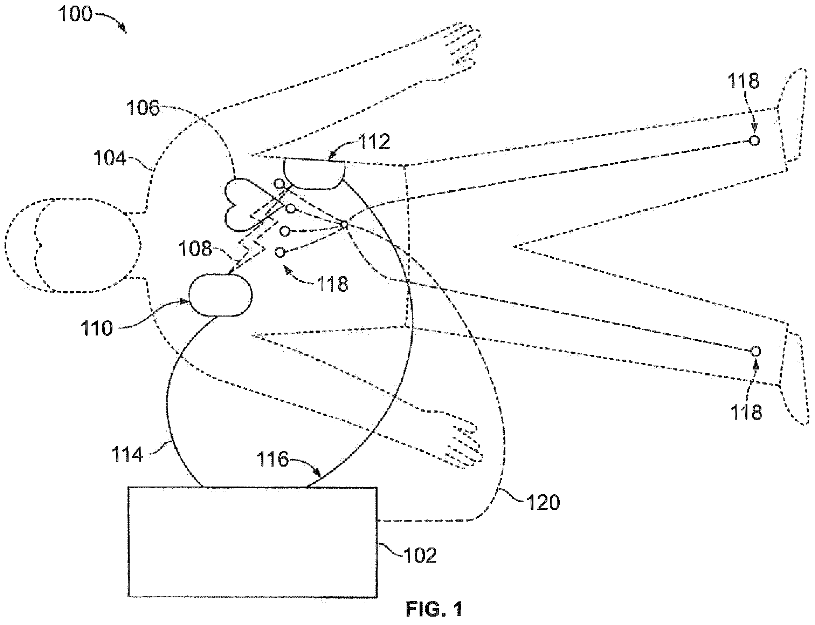

Particular embodiments are described herein with reference to the drawings. In the description, common features may be designated by common reference numbers throughout the drawings. In some drawings, multiple instances of a particular type of feature may be used. Although these features are physically and/or logically distinct, the same reference number is used for each, and the different instances are distinguished by addition of a letter to the reference number. When the features as a group or a type are referred to herein (*e.g.*, when no particular one of the features is being referenced), the reference number is used without a distinguishing letter. However, when one particular feature of multiple features of the same type is referred to herein, the reference number is used with the distinguishing letter. For example, referring to FIG. 1, sensors are illustrated and associated with reference number 118. When referring to a particular one of the sensors, such as the sensor 118A, the distinguishing letter "A" may be used. However, when referring to any arbitrary one of the sensor or to the sensors as a group, the reference number 118 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (*e.g.*, "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

The present application is directed to embodiments that relate to systems, methods, and apparatus to provide a real-time audio feedback for use during administration of an emergency medical treatment of a patient by medical personnel. During an emergency episode, the embodiments may provide audio fonts to alert a user or rescuer to respond to medical situations. This can include performing appropriate emergency procedures, such as CPR treatments, on a person or patient. Also, for example, audio fonts can guide medical personnel in adjusting performance of a procedure (e.g. the audio font is communicating detected deviation away from an optimal state, such as attainment of full recoil after each chest compression, and so forth, thereby allowing the rescuer to adjust back towards the optimal state). As another example, audio fonts can help with pausing or stopping performance of a procedure, altering/adjusting which procedure to perform next, or when it might be the appropriate/necessary time to switch procedures (*e.g.* to switch from a continued attempt at intubation, to halting the intubation attempt and begin attempting to reoxygenate the patient in preparation for a next attempt at intubation).

Audio fonts can also be used as an alert for degraded reliability of patient data, such as physiologic data (*e.g.* because signal quality has degraded), and as an alert to passage of time. For example, a CPR metronome may be configured with several audio fonts, and the metronome tones can be configured to switch between those audio fonts at pre-determined configurable times, such that the audio font serves as a cue to the team of rescuers of where they are in the 2-minute CPR cycle. For example, font "A" could be used for the 1st minute, font "B" could be used for the next 30 seconds, font "C" could be used for the next 15 seconds, and font "D" could be used for the final 15 seconds. Since for many resuscitation teams, each provider has a different task or set of tasks to perform, at specific points during the 2 minute cycle, the audio font can communicate (without the extraneous sound and cognitive clutter of *e.g.* verbal prompts, or needing to watch a clock) which phase of the 2-minute cycle they are in, so that they know when it is time to perform their task. Tasks may include, for example, delivering a medication (sometimes done half way through a 2-minute cycle), checking a rhythm or checking for a pulse, preparatory positioning of the next provider who will take over performing chest compressions for the next 2-minute cycle, charging the defibrillator, or preparing to make a physiologic measurement or assessment at the end of the 2-minute cycle (*e.g.* a provider positioning their fingers, or a Doppler probe, *e.g.* on a femoral artery, to be able to pick up the pulse from the last several chest compressions, and then rapidly determine if pulses continue to be sensed after cessation of chest compressions, indicating ROSC). In current practice, there often is one individual assigned to be the "time keeper" of the resuscitation, and they typically may call out these time points to the team, to facilitate the type of team coordination and sequencing described above. However, an audio font may be used as a substitute for the "time keeper" individual, providing the same kind of awareness of the progression through the 2-minute cycle, with distinct fonts for each phase that are associated with specific team actions. In other words, the audio font can serve as a team coordination tool, that can replace the need for a dedicated individual to perform that function, and also replace the need for the entire team to be able to see and repeatedly check a visual coordination tool (such as a clock display, or some other visual "progress of time" display).

Awareness that the monitored signals or measurements have crossed a critical threshold may alter real-time care and treatment decisions by medical personnel. Another application of the audio fonts can be to indicate signal quality for incoming patient data. A further application of the audio fonts can be to indicate the passage of time within a procedure or care process.

Referring now to the figures, FIG. 1 is a diagram of a representation of an exemplary scene 100 showing use of a medical device 102 for monitoring and providing treatment or therapy to a person 104 experiencing a medical condition, such as cardiac arrest. The medical device 102 may be operated by a user (*e.g.,* a healthcare professional, service worker, a doctor, a first responder, etc.) and may be used in a hospital or a pre-hospital environment or setting. The medical device 102 may include functions and operations of an external defibrillator, such as a manual defibrillator, an automatic external defibrillator (AED), or any other suitable defibrillator. In some examples, the medical device 102 may be a monitor defibrillator, which is a combination of a monitor and a defibrillator.

As shown in FIG. 1, the medical device 102 is positioned near the person 104 (*e.g.* a patient). For illustrative purposes, and not meant to be a limiting example, the person 104 may be a patient with a severe head injury (*e.g.*, without heart related conditions, not in cardiac arrest, etc.) and in whom the emergency providers may be performing an endotracheal intubation (*e.g.* an RSI). In this scenario, there may be no defibrillation shocks, no CPR, no use of AEDs, no defibrillation electrodes placed on the patient. However, similar to the CPR situation illustrated herein, this is another example of a "lifesaving intervention." Audio fonts may be used to aid in, and/or signal progression of, the various responses by medical personnel to the condition of person 104.

In a CPR situation, the person 104 may be experiencing a heart condition in heart 106, which could be, for example, cardiac arrest or any other cardiac rhythm abnormality. The person 104 may be lying on their back on a surface, such as the ground or a bed, and may be located in a hospital, a home, or a pre-hospital environment (*e.g.*, an ambulance). The medical device 102 may be configured to generate an electrical pulse 108 and deliver the electrical pulse 108 to the person 104. The electrical pulse 108, also known as a defibrillation shock or therapy shock, is intended to go through and restart the heart 106, in an effort to save the life of the person 104. The electrical pulse 108 can further include one or more pacing pulses and the like.

The electrical pulse 108 may be delivered to the person 104 using defibrillation electrodes 110 and 112. The defibrillation electrodes 110 and 112 may include hand-held electrode paddles or electrode pads placed on the body of the person 104 (*e.g.,* the chest of the person 104). An electrical cable 114 may connect the defibrillation electrode 110 to the medical device 102 and an electrical cable 116 may connection the defibrillation electrode 112 to the medical device 102. When the defibrillation electrodes 110 and 112 are in electrical contact with the body of person 104, the medical device 102 may administer, via the defibrillation electrodes 110 and 112, the electrical pulse 108 through the heart 106 of the person 104. The defibrillation electrodes 110 and 112 may also be configured to sense or detect one or more physiological parameters of the person 104 and to generate signals representative of the physiological parameters.

As shown in phantom in FIG. 1, one or more sensors or electrodes 118 may be placed at various locations on the body of the person 104. The sensors 118 may be configured to sense or detect physiological parameters of the person 104 and to produce signals representative of the physiological parameters. An electrical cable 120 may connect the sensors 118 to the medical device 102. The physiological parameters generated by the sensors 118 and/or the defibrillation electrodes 110 and 112 may be provided to the medical device 102 for evaluation and analysis. The physiological parameters may include $SpO_2$ measurements, ECG data, heart rhythm data, heart rate data, cardiac output data, blood flow data, a level of perfusion, respiration data, body temperature data, and/or any other suitable physiological parameter that may be used to assess the physical condition of the person 104.

The medical device 102 may include a user interface having an input device configured to receive input or selections from a user or rescuer for displaying information related to vital signs for the person 104. The displayed information may relate to pulse oximetry ($SpO_2$), carboxyhemoglobin (SpCO), Methemoglobin (SpMet™), non-invasive blood pressure (NIBP), heart rate (HR), and/or end tidal $CO_2$ (ETCO2) current values, blood pressure (BP) measurements, among others. The user interface may allow the user to input or select and/or configure various operational parameters for medical device 102. In some implementations, the medical device 102 may be automatically configured, upon activation or set-up, for an adult with an unsecured airway. The user interface of the medical device 102 may also allow a user to request or specify a time period for a cardiac or resuscitation treatment. For example, the user may input or select a CPR time period for performing a CPR treatment. In some implementations, the user may input or select a CPR time period from one or more preset or predetermined CPR periods (*e.g.,* 15 seconds, 1 minute, 2 minutes, 3 minutes, etc.). Generally speaking, medical device 102 may be configured for multi-parameter monitoring, Shock Advisory, manual and automatic defibrillation, synchronized cardioversion, pacing, and wireless post event record transfer.

For example, the medical device 102 may be configured to select a CPR treatment protocol for delivery of a CPR treatment to the person 104 based on the user inputs and/or physiological parameters of the person 104. Also, for example, the medical device 102 may identify and retrieve a CPR treatment protocol from memory based on the inputs from a user. The CPR treatment protocol may include a pattern or ratio of a number of chest compressions to one or more ventilations for a CPR cycle of the CPR treatment *(e.g.,* a number of chest compressions to be delivered followed by one or more ventilations during a CPR cycle). The medical device 102 may modify or adjust the selected CPR treatment protocol or the CPR time period inputted or selected by a user for the CPR treatment as further described below.

The user interface of the medical device 102 may also include output devices to provide visual or aural signals to the user. For example, the output devices may provide signals and prompts to assist a user or rescuer in delivering a cardiac or resuscitation treatment, such as a CPR treatment, to the person 104. The output devices of the user interface may display representations of physiological parameters to assist a user in treating and diagnosing medical conditions of the person 104. Additionally, the output devices may provide instructions to a user for delivering a defibrillation pulse to the person 104.

In some embodiments, the output devices may play, to an environment of the medical device 102, an audio signal indicating an audible change in a particular audio font. The audible change reflects whether a particular characteristic of the measurement data has crossed a clinically meaningful threshold. This can facilitate a user to administer the appropriate medical procedures to the person 104. Generally, clinically meaningful thresholds may depend on a type of measurement data, a type of medical device 102, and/or a particular health condition of a patient such as person 104.

In the context of an oxygen saturation (SpO2) monitor, the output devices may play sounding tones corresponding to photoplethysmographic peaks that can be used to convey information about the patient's pulse rate. SpO2 values indicate an amount of oxygen in the blood as a percentage of a maximum capacity in the blood. SpO2 values may be conveyed by changing the tone pitch with respect to those SpO2 values displayed on the monitor. Both the pitch and the audio font of the SpO2 tones represent sonifications of physiologic and device information. Audible monitoring enhances patient care by conveying useful information without demanding attention to the device display.

As described herein, in some embodiments, the audio fonts may be used as a continuously changing parameter, rather than just one that makes a step change at one or more critical thresholds. For example, similar to mapping pitch to SpO2 as a continuous function, the audio font may be mapped to signal quality (or some other signal characteristic) in a continuously varying way. In some embodiments, a multiple critical threshold scheme may be viewed as a discrete example of such a "continuously-varying" case, since multiple thresholds are "coarsely varying via multiple discrete steps."

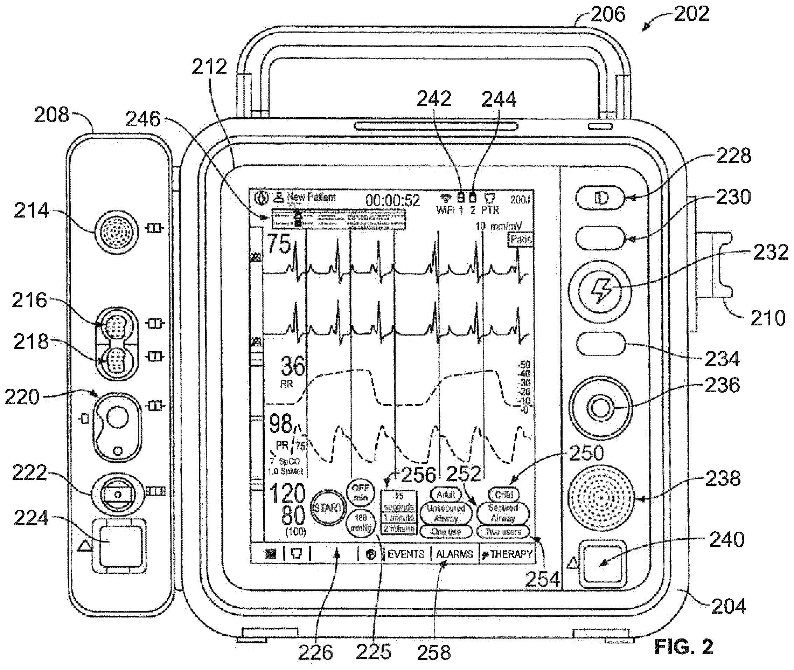

FIG. 2 illustrates a front view of a medical device 202, in accordance with an example implementation. The medical device 202 can comprise the medical device 102 of FIG. 1. The medical device 202 may be configured to monitor and provide treatment or care to a person or patient experiencing a medical condition, such as cardiac arrest or any other cardiac rhythm abnormality. The medical device 202 may be operated by a user *(e.g.,* a medical professional, a first responder, a healthcare professional, service worker, a doctor, etc.) and may be used in a hospital or a pre-hospital environment or setting. As illustrated, the medical device 202 may be a monitor defibrillator, which is a combination of a monitor and a defibrillator.

As a defibrillator, the medical device 202 may be configured to deliver an electrical pulse or shock to a person experiencing a medical condition. The medical device 202 may be configured to operate or function in one or more defibrillation modes, such as a manual defibrillation mode, an AED mode, or any other suitable defibrillation mode. For example, the medical device 202 may be selected to operate in an AED mode when the medical device 202 is intended to be used by a first responder and/or a layperson who may have no or minimal training in providing medical treatment using defibrillation. When operating in the AED mode, the medical device 202 may determine whether a defibrillation pulse or shock is needed and, if so, charge an energy storage device *(e.g.,* a capacitor) of the medical device 202 to a predetermined energy level and instruct the user to administer the defibrillation shock. The medical device 202 may also be selected to operate in a manual defibrillation mode when the medical device 202 is intended to be used by persons who are trained to provide medical treatment using defibrillation (*e.g.*, medical professionals, such as doctors, nurses, paramedics, emergency medical technicians, etc.). When the medical device 202 is configured to operate in the manual defibrillation mode, the user may determine whether a defibrillation pulse or shock is needed and may control the administration of the defibrillation shock to the person.

As a monitor, the medical device 202 may monitor and evaluate physiological parameters of a person being treated for a medical condition. The medical device 202 may receive signals or voltages from one or more electrodes or sensors placed at various locations on the body of the person. The electrodes or sensors may sense or detect the physiological parameters of the person and produce signals representative of the physiological parameters. The representations of the physiological parameters may be displayed by the medical device 202 to assist a user in treating and diagnosing medical conditions of a person. The physiological parameters may include ECG (electrocardiogram) data, body temperature, non-invasive blood pressure (NIBP), arterial oxygen saturation/pulse oximetry (SpO2), a concentration or partial pressure of carbon dioxide in the respiratory gases *(e.g.,* capnography), and/or any other suitable physiological parameter of a person. The physiological parameters of the person can be stored in the memory of the medical device 202.

In some examples, the physiological parameters may be transmitted to other remote communication or computing devices and databases.

Aspects of the treatment being provided may include a depth or recoil amount of a chest compression, or a parameter reflective of the adequacy/security of an airway that has been established to oxygenate and ventilate a patient (such as adequate mask seal). Aspects of the environment may include motion (*e.g.* vehicle motion or patient motion). Actions and/or status of the provider may include detection of fatigue during performance of chest compressions (*e.g.* an algorithm may integrate various dimensions of measured chest compression quality into an index indicative of potential fatigue, and the algorithm may be configured to alert the team to this developing fatigue via an audio font, allowing perception of the information without the need to see and/or watch a monitor screen, or experience additional sonic/cognitive clutter from voice prompts or similar warnings delivered via different modalities), or audio-font-mediated feedback on the progress of an intubation attempt wherein a video laryngoscope is being used to execute the procedure, and a real-time algorithm (*e.g.* a computer-vision AI algorithm) is being run on the video data, generating real-time feedback on the provider's navigation of the tip of the laryngoscope blade through the succession of anatomic reference points or waypoints towards obtaining visualization of the vocal cords. For example, in the event the user is wandering "off course" as determined by the algorithm, the audio font can change (in either a binary or proportional manner) to communicate deviation from the apparent optimal course as determined by this "decision assist" feature.

As shown in FIG. 2, the medical device 202 includes a housing or casing 204, a handle 206, an input module or interface 208, a defibrillation interface 210, and a user interface 212. The handle 206 of the medical device 202 is attached to the housing 204 to allow a user to move or transport the medical device 202 to a location near a person experiencing a medical condition. The housing 204 may be generally rectangular or square shaped. In other examples, the housing 204 can have any other suitable shape. The housing 204 may be formed from various materials or combinations of materials. For example, the housing 204 may be made of molded plastic, metal, or some combination of both.

The input module 208 of the medical device 202 may be coupled to or integral with the housing 204. The input module 208 may enable the medical device 202 to receive physiological parameters (*e.g.*, a heart rate (HR) and ECG data) of a person via sensors (*e.g.*, the sensors 118 of FIG. 1). The sensors may be placed on the body of a person to sense or detect signals generated by the body or heart of the person. The input module 208 may include one or more ports or receptacles to enable the sensors to be detachably coupled to the input module 208. As shown in FIG. 2, the input module 208 can include a port 214 configured to be connected to an oxygen saturation (SpO2) sensor, a port 216 configured to be connected to a temperature sensor, a port 218 configured to be connected to a sensor for measuring invasive blood pressure (IP) via a catheter, a port 220 configured to be connected to a sensor for measuring partial pressure of carbon dioxide (CO2) in gases in the airway via capnography, and a port 222 configured to be connected to a sensor for measuring a non-invasive blood pressure (NIBP). The input module 208 may also include a communication port 224, such as a Universal Serial Bus (USB) port, that can be used to connect to an input device *(e.g.,* a mouse, a keyboard, etc.). The input module 208 may include other ports to enable any other suitable physiologic parameter of a person to be monitored and evaluated by the medical device 202.

The defibrillation interface 210 of the medical device 202 may be configured to enable electrical charges or pulses to be delivered or applied to a person via defibrillation electrodes (*e.g.*, the defibrillation electrodes 110 and 112 shown in FIG. 1). The defibrillation electrodes may also be configured to enable the medical device 202 to receive physiological parameters of a person (*e.g.,* a heart rate (HR), ECG data, etc.). The defibrillation interface 210 may include one or more ports or receptacles capable of allowing the defibrillation electrodes to be detachably coupled to the defibrillation interface 210. A cable assembly or therapy cable may enable the defibrillation electrodes to be coupled to the ports of the defibrillation interface 210. Each therapy cable may include a defibrillation electrode attached at one end and a connector attached to the other end. The connector may be configured to be coupled to or plugged into a port or receptacle of the defibrillation interface 210.

The user interface 212 of the medical device 202 may include one or more input devices configured to receive inputs or commands from a user and one or more output devices configured to provide information to the user. The input devices of the user interface may include various controls, such as switches, pushbuttons, keyboards, touchscreens, microphones, scanners, and/or cameras, etc., for operating the medical device 202. For example, the input devices may be configured to receive user inputs related to the pitch and the audio font of the SpO2 tones that represent sonifications of physiologic and device information. The output devices of the user interface 212 can be visual, audible or tactile, for communicating to a user of the medical device 202 *(e.g.,* a medical professional, a first responder, etc.). For example, the output devices may be configured to present visual alarms or alerts, flashing lights, and/or warnings to the user. The output devices may also include an audio system that provides audio signals to aurally communicate with the user voice prompts that deliver instructions or commands, monotonal, ascending, descending or quickening tones to indicate alerts or warnings, and/or any other suitable audio signals for communicating with the user of the medical device 202.

As shown in FIG. 2, the user interface 212 of the medical device 202 includes a power button 228 configured to turn the medical device 202 on and off *(e.g.,* "On-Off" button), a charge button 230 configured to cause the medical device 202 to build an electric charge to be applied to the person in a form of a defibrillation shock or pulse, a defibrillation shock button 232 configured to cause the medical device 202 to apply a defibrillation pulse or therapy shock to a person during a defibrillation episode, an analyze button 234 configured to cause the medical device 202 to analyze physiologic parameters of a person (e.g., ECG data) to facilitate determining the appropriate time to apply a defibrillation pulse or shock, and a speed dial button 236 configured to navigate through menus of on-screen software. The user interface 212 may also include a speaker 238 to provide audio output and a USB output port 240 to facilitate connecting the medical device 202 to a device, such as a printer. The user interface 212 may include any other suitable output device for providing outputs or input device for receiving inputs from a user.

In some embodiments, the medical device 202 may be configured to display a graphical user interface (GUI) 226 that includes graphical elements representative of one or more aspects of analyzed measurement data. For example, the user interface 212 of the medical device 202 may include a display device 225 (e.g., a touchscreen) for displaying GUI 226. The GUI 226 may display physiologic parameters of a person (e.g., a patient), provide visual feedback to the user (e.g., a healthcare provider) about a condition of the person, provide information about the medical device 202 (e.g., battery information), and allow the user to interact with and operate the medical device 202. The GUI 226 may also be configured to visually present various measured or calculated parameters associated with the person indicating the physical status of the person (e.g., an ECG, SpO2 values, SpCO values, SpMet™ values, NIBP, HR, BP measurements, and/or ETCO2 current values). Further, the GUI 226 may provide instructions and/or commands, including prompts to perform cardiopulmonary resuscitation (CPR) treatment or other treatment, to the user. Additionally, the GUI 226 may include multiple visual user interface items that are selectable or "clickable" by the user including user-selectable icons, user-selectable on-screen buttons, menus, widgets, scroll bars, and graphical objects, and/or other items for facilitating user interaction.

As shown in FIG. 2, the GUI 226 includes graphical representations of a first battery unit indicator 242 and a second battery unit indicator 244. The first battery unit indicator 242 and second battery unit indicator 244 are configured to display a status of a first battery unit and a status of a second battery unit, respectively, of the medical device 202. For example, the first and second battery unit indicators 242 and 244 may provide a charging level of the first and second battery units.

The GUI 226 may also allow a user or rescuer to input information (e.g., parameters, variables, etc.) for enabling the medical device 202 to identify and select a cardiac or resuscitation treatment protocol (e.g., a CPR treatment protocol) for a person experiencing a medical condition, such as cardiac arrest. For example, the GUI 226 may allow the user to input an airway status (e.g., whether the person has their airway secured by artificial airway), whether the CPR is being delivered by one or two persons, whether the person delivering CPR is a medical professional or a layperson, parameters associated with the pitch, intensity and the audio font of the tones that represent sonifications of physiologic and device information, and/or other variables which may be used by the medical device to determine or select a CPR treatment protocol. The GUI 226 may enable a user to input the information through user-selectable on-screen graphical items. In some implementations, the user interface may include the following user-selectable user-interface items: "Adult" and "infant/child" 250, "secured airway" and "unsecured airway" 252, "one provider" and "two providers" 254, and alarms 258. The user-selectable user-interface items may allow the user to provide input by pressing or selecting areas on the GUI 226 displaying the items.

The GUI 226 of the medical device 202 may also allow a user to input a time period for performing a cardiac or resuscitation treatment, such as a CPR time period for performing a CPR treatment. For example, the GUI 226 may allow a user to input or select a CPR time period for performing a CPR treatment from preset or predetermined time limits. The GUI 226 may display user-selectable user-interface items 256 to allow a user to input or select the CPR time periods. In some implementation, the GUI 226 may include the following user-selectable user-interface items: "15 seconds", "1 minute, "2 minutes", and "3 minutes".

The GUI 226 may provide signals and prompts to assist a user or rescuer for delivering the medical treatment in accordance with modified sonifications. For example, the GUI 226 of the medical device 202 may provide distinct timbral and intensity signatures that may be applied at clinically-meaningful measurement thresholds (e.g., SpO2 value thresholds) to more clearly indicate to a user that measurement values (e.g., SpO2 values) are not only changing, but have crossed a critical threshold.

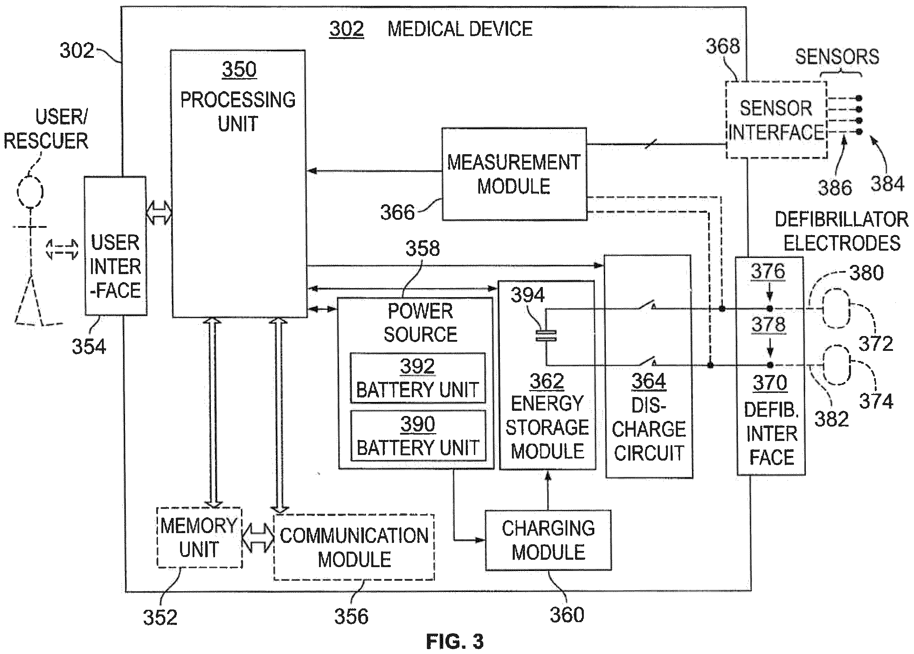

FIG. 3 is a diagram showing components of a medical device 302, in accordance with an exemplary embodiment. As shown in FIG. 3, the medical device 302 includes a processing unit 350, a memory unit 352, a user interface 354, a communication module or interface 356, a power source 358, a charging module 360, an energy storage module or device 362, a discharge circuit 364, a measurement module 366, a sensor interface 368, and a defibrillator interface 370. These components can be, for example, included in the medical devices of Figures 1 or 2.

The defibrillation interface 370 of the medical device 302 may be configured to enable an electrical pulse or shock to be delivered or applied to a person (e.g., a patient) experiencing a medical condition. The defibrillation interface 370 may comprise the defibrillation interface 210 of FIG. 2. The defibrillation interface 370 may include one or more ports or nodes 376 and 378 to enable the defibrillation electrodes 372 and 374 to be detachably coupled to the defibrillation interface 370. A cable assembly or therapy cable 380 may enable the defibrillation electrode 372 to be coupled to the port 376 of the defibrillation interface 370 and a cable assembly or therapy cable 382 may enable the defibrillation electrode 374 to be coupled to the port 378 of the defibrillation interface 370 of the medical device 302. The cable assembly 380 may include the defibrillation electrode 372 attached at one end and a connector attached to the other end, and the cable assembly 380 may include the defibrillation electrode 374 attached at one end and a connector attached to the other end. The connectors of the cable assemblies 380 and 382 may be configured to be coupled to or plugged into the ports 376 and 378 of the defibrillation interface 370. The connectors can be male or female connectors that are compatible with the ports 376 and 378 of the defibrillation interface 370 of the medical device 302.

The defibrillation interface 370 may also enable the medical device 302 to receive physiological parameters (*e.g.*, HR, ECG) of the person or patient. For example, each of the defibrillation electrodes 372 and 374 may be configured to measure one or more physiological parameters of the person (*e.g.*, heart electrical activity, heart rate, etc.) and to provide signals representative of the physiological parameters to the defibrillation interface 370. For example, when the defibrillation electrodes 372 and 374 are placed on the chest of the person, an ECG signal of the person can be detected as a voltage difference between the defibrillation electrodes 372 and 374. The defibrillation electrodes 372 and 374 may also be used to determine device parameters indicative of a condition of the medical device 302, such as an electrode impedance or a user interaction with the medical device 302. For example, the medical device 302 can detect an impedance between the defibrillation electrodes 372 and 374 to determine whether the defibrillation electrodes 372 and 374 are making sufficient electrical contact with a person's body.

The sensor interface 368 of the medical device 302 may be configured to receive physiological parameters (*e.g.*, a heart rate (HR) and ECG data) from one or more sensors 384 (*e.g.*, physiological sensors). The sensors 384 may be placed in contact with the body of a person being monitored or treated for a medical condition. Each of the sensors 384 may include a transducer configured to sense a signal or voltage of the person. For example, the sensors 384 may measure or detect heart electrical activity and other parameters, such as an electrocardiogram (ECG), saturation of the hemoglobin in arterial blood with oxygen (SpO2), carbon monoxide (carboxyhemoglobin, COHb) and/or methemoglobin (SpMet™), partial pressure of carbon dioxide ($CO_2$) in gases in the airway by means of capnography, total air pressure in the airway, flow rate or volume of air moving in and out of the airway, blood flow, blood pressure (*e.g.*, non-invasive blood pressure (NIBP)), core body temperature with a temperature probe in the esophagus, oxygenation of hemoglobin within a volume of tissue (rSO2), and any other physiological parameters of a person being monitored or treated.

The sensor interface 368 may include one or more receptacles or ports (*e.g.,* an ECG port) to enable the sensors 384 to be detachably coupled to the medical device 302. The sensors 384 may be attached to the sensor interface 368 by cable assembles or therapy cables 386. In some embodiments, the sensors 384 may be fixedly connected to the sensor interface 368. The therapy cables 386 may each include a sensor 384 at one end and a connector at the opposite end. The connector can be configured to be coupled to or plugged into a port or receptacle of the sensor interface 368.

The measurement module 366 of the medical device 302 may be configured to receive signals or sensor data from the sensor interface 368 and the defibrillation interface 370. The signals may be representative of physiological parameters or conditions of a person being monitored and/or treated for a medical condition. The measurement module 366 may measure or determine various physiological parameters from the signals. For example, the measurement module 366 may determine an ECG of the person based on a voltage difference between the defibrillation electrodes 372 and 374. The measurement module 366 may also determine device parameters indicative of a condition of the medical device 302, such as an electrode impedance or a user interaction with the medical device 302. For example, the measurement module 366 may measure an impedance or voltage across the defibrillation electrodes 372 and 374 or a pair of sensors 384.

The measurement module 366 may also include a filter circuit or hardware (*e.g.*, amplifiers, filters, etc.) to attenuate and/or filter at least some of the noise that may be present on the signals received from the sensor interface 368 and/or the defibrillation interface 370. For example, the filter circuit may apply at least one filter to the signal to remove artifacts resulting from chest compressions being delivered to the person. In some embodiments, the filter may be implemented as an analog filter, a digital filter, or combinations of both. The measurement module 366 may also digitize the signals received from the sensor interface 368 and/or defibrillation interface 370 prior to transmitting the signals to the processing unit 350.

The user interface 354 of the medical device 302 may facilitate user interactions with the medical device 302. The user interface 314 may comprise the GUI 226 of FIG. 2. The user interface 354 may include various types of input devices for receiving inputs or commands from a user. For example, the input devices may include keyboards, switches, microphones, pushbuttons, touchscreens, scanners, and/or any other suitable input device for enabling inputs to the medical device 302. For instance, a user can use the input devices on the user interface 354 to input information regarding a particular event (*e.g.,* a treatment or medication administered to the person).

The input devices of the user interface 354 may also allow a user or rescuer to input information (*e.g.*, parameters, variables, etc.) for enabling the medical device 302 to emit distinct timbral and/or intensity signatures that may be applied at clinically-meaningful measurement thresholds (*e.g.*, SpO2 value thresholds) to more clearly indicate to a user that measurement values (*e.g.*, SpO2 values) are not only changing, but have crossed a critical threshold for a person experiencing a medical condition, such as cardiac arrest. For example, the user devices of the user interface 354 may allow the user to input or select an age classification of a person (*e.g.,* adult or child/infant), an airway status (*e.g.,* whether the person has their airway secured by artificial airway), whether the CPR is being delivered by one or two persons, whether the person delivering CPR is a medical professional or a layperson, and values for pitch, timbre and intensity, and/or other variables which may be used by the medical device 102 to select and/or deliver a treatment protocol. In some implementations, the medical device 302 may be automatically configured, upon activation or set-up, for an adult with an unsecured airway.

The input devices of the user interface 354 may also allow the user to input or select a time period for performing a cardiac or resuscitation treatment, such as a CPR time period for performing a CPR treatment. In some implementations, the user may select or input a CPR period for the CPR treatment from preset or predetermined time limits (*e.g.,* 15 seconds, 1 minute, 2 minutes, 3 minutes, etc.). Once the user inputs or selects the CPR time period for the CPR treatment, the medical device 302 may determine or select, based on the user inputs, a CPR treatment protocol from memory (*e.g.,* the memory unit 352). The medical device 302 may evaluate the CPR treatment protocol for the CPR treatment. Based on the evaluation, the medical device 302 may modify the CPR treatment protocol and/or may modify or adjust the CPR time period inputted or selected by the user for the CPR treatment as further described below.

The user interface 354 may also comprise various types of output devices for providing information to the user. For example, the output devices may provide instructions or prompts to assist a user or rescuer for delivering CPR treatments to a person experiencing a medical condition, such as cardiac arrest. The output devices of the user interface can be visual, audible or tactile for communicating to or providing feedback to a user (*e.g.* a rescuer, a first responder, a healthcare professional, etc.). The output devices may include a screen, a display, one or more light emitting diodes (LEDs), and/or a speaker to output various sounds (*e.g.*, voice or audio), etc. The output devices may be configured to present visual alarms or alerts, flashing lights, and/or warnings to the user of the medical device 302. The output devices may also include an audio system that provides an audio signal to aurally communicate with user voice prompts that deliver instructions or commands, monotonal, ascending, descending or quickening tones to indicate alerts or warnings, and/or any other suitable output device for communicating with the user.

Further, the output devices of the user interface 354 may include a metronome or a metronome system for providing signals to guide the user in pacing and timing of chest compressions for cardiac or resuscitation treatments (*e.g.*, a CPR treatment) and, in some implementations, for providing signals to guide in the pacing and timing of ventilations for the a cardiac or resuscitation treatment. The signals may be visual (such as flashing lights or graphics on a display screen), or may he aural. In some embodiments, the device may include one or more measurement modes, wherein each measurement mode of the one or more measurement modes is associated with a corresponding range of measurement values. Such embodiments may involve mapping, for each measurement mode, the corresponding range of measurement values in terms of the intervals of musical notes, identifying, for each measurement mode, a plurality of intervals for the corresponding range of measurement values, and associating, for each measurement mode, a respective plurality of audible fonts with the corresponding plurality of intervals.

For example, the metronome may deliver to the user a first type of signal for chest compressions, a second type of signal for ventilations, and a third type of signals to indicate an upcoming transition from compressions to ventilations (or, in a protocol where ventilations are given without a pause in compressions, to indicate an upcoming ventilation series). The different signal types may be distinguishable from one another by the user. When flashing lights are used, different colors may distinguish between compressions, transitions, and ventilations. The aural signals may be any of a variety of sounds such as tones, beeps, tocks, clicks, and the like, or may be voiced (for example, "press-press-press" for compressions, "ventilate" or "blow" for ventilations). In some embodiments, a user may choose whether to have the metronome deliver voiced or non-voiced sounds (*e.g.*, tones, beeps, clicks, tocks, or other non-verbal sounds) through a set-up menu upon device set-up.

The signals for chest compressions may be rhythmic signals such as a series of identical sounds delivered at a rate corresponding to the desired rate for chest compressions. A sound that is suggestive of or approximates the sound of ventilation may be used for a ventilation signal. The sound signal used for each ventilation may have a duration that corresponds to the desired duration of the ventilation. For a ventilation series having more than one ventilation, the ventilation sound signals may also be delivered at a rate equal to the desired rate for ventilation delivery. The transition signals may advise the user that a transition from chest compressions to ventilations (with or without a pause in chest compressions) is coming up soon. For example, where tones, beeps, clicks or tocks are used to indicate chest compressions for a cardiac or resuscitation treatment (*e.g.*, 30:2 compressions to ventilations protocol for a CPR treatment protocol), the transition signal may be a voiced countdown of the last few compressions in a series.

Further, the output devices of the user interface 352 may include a display configured to visually present to the user various measured or calculated parameters associated with a person or patient experiencing a medical condition, such as cardiac arrest. For example, the display can be configured to visually present an ECG and/or other physiological signals indicating the physical status of the person in cardiac arrest. The output devices may also provide instructions and/or commands, including prompts to perform CPR treatments or other treatments, to the user.

The memory unit 352 of the medical device 302 may be in operable communication with the processing unit 350. The memory unit 352 may store various values, look-up tables, equations, audio and video files, and/or a plurality of audio font protocols that can be read and accessed by the processing unit 350. The audio font protocols may vary depending on factors such as age classification of the person experiencing a medical condition (*e.g.*, adult or child/infant), an airway status (*e.g.*, whether the person has their airway secured by artificial airway) or other physiologic specific factors that may affect optimal oxygenation levels or thresholds.

Further, the memory unit 352 can store instructions or computer executable code of software routines that can be retrieved and executed by the processing unit 350. The memory unit 352 may include one or more computer-readable storage media that can be read or accessed by the processing unit 350. The computer-readable storage media can include volatile and/or non-volatile memory, dynamic random-access memory (DRAM) read-only memory (ROM), random access memory (RAM), magnetic disk storage media, optical storage media, smart cards, flash memory devices, or any other suitable memory. In some embodiments, the computer-readable storage media can be integrated in whole or in part with the processing unit 350. The computer-readable storage media may be implemented using a single physical device (*e.g.,* one optical, magnetic, organic or other memory or disc storage unit), while in other examples, the computer readable storage media can be implemented using two or more physical devices.

The processing unit 350 of the medical device 302 may be configured to control various operations of the medical device 302. The processing unit 350 may receive inputs from the user and various components of the medical device 302 and may process the inputs to produce outputs that may be stored in the memory unit 352 and/or displayed or communicated via the user interface 354. For example, the processing unit 350 may be configured to perform operations. The operations may include analyzing, by a medical device, measurement data collected from the patient. The operations may also include associating, by the medical device, a particular audio font based on a characteristic of the measurement data. The operations may further include identifying, by the medical device and based on the characteristic of the measurement data, one or more clinically meaningful thresholds in the measurement data. The operations may also include generating, by the medical device, an audible change in the particular audio font to reflect whether a particular characteristic of the measurement data has crossed a clinically meaningful threshold of the one or more clinically-meaningful thresholds.

To illustrate further in the context of a SpO2 monitor, processing unit 350 of the medical device 302 may be configured to generate sounding tones corresponding to photoplethysmographic peaks that can be used convey information about the patient's pulse rate. The processing unit 350 of the medical device 302 may be configured to convey SpO2 values by changing the tone pitch with respect to those SpO2 values displayed on the user interface. Both the pitch and the audio font of the SpO2 tones represent sonifications of physiologic and device information. Audible monitoring enhances patient care by conveying useful information without demanding attention to the device display. In order to be useful, clear discrimination of the audible information is needed. Generally, tone pitches may be substantially similar in frequency, thereby resulting in an inability to differentiate between SpO2 values.

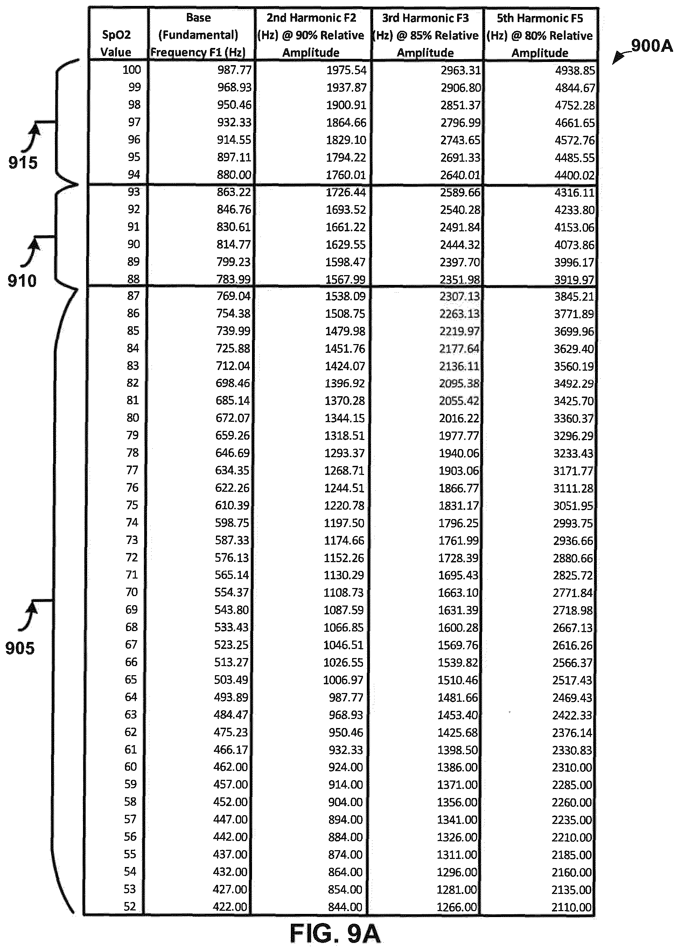

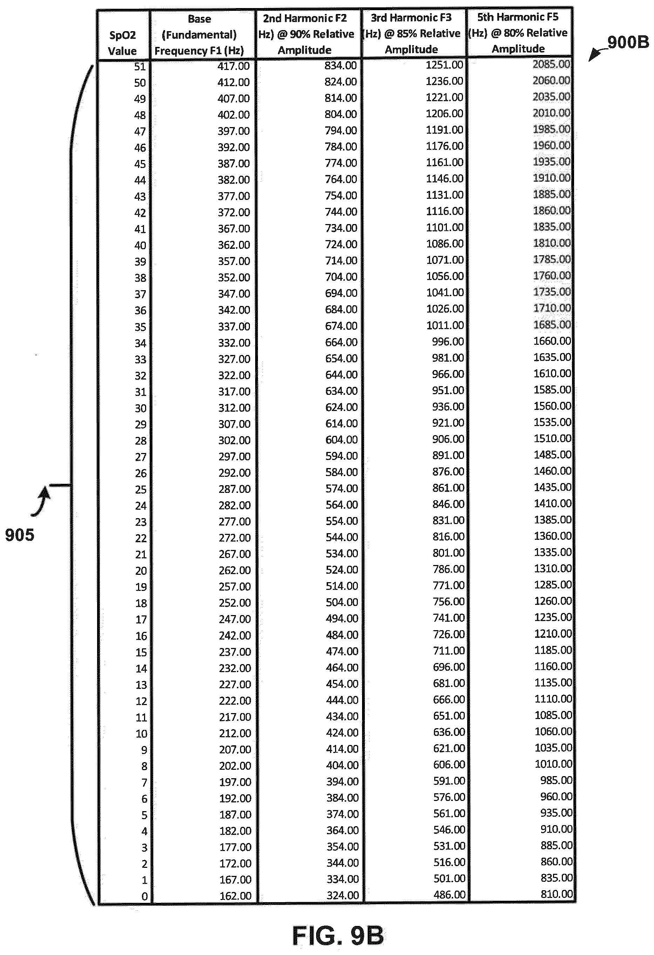

For example, the processing unit 340 may determine from tables or information stored in the memory unit (*e.g.,* table 900A of FIG. 9A and table 900B of FIG. 9B) associating a plurality of audible fonts with the corresponding plurality of identified intervals. The one or more clinically meaningful thresholds may include a transition of a SpO2 value from a given interval of the plurality of intervals to a next interval of the plurality of intervals. For example, the plurality of intervals may include a first interval of SpO2 values from 100% to 94%, a second interval of SpO2 values from 93% to 88%, and a third interval of SpO2 values from 87% to 0%. The plurality of audible fonts may include a first audio font corresponding to the first interval, a second audio font corresponding to the second interval, and a third audio font corresponding to the third interval, and wherein the one or more clinically meaningful thresholds comprises 94% or 88%.

The processing unit 350 may also cause an electrocardiogram (ECG) to be displayed on the user interface 354 that reflects the sensed electrical activity of a heart of a person. The processing unit 350 may analyze the ECG data through an algorithm to detect the R-Wave (QRS Complex) for all connected ECG Leads Channels, including QRS detection, Heart Rate Calculation, and Heart Rate. The processing unit 350 may also cause waveform representation of the ECG signals to be displayed on the user interface 354. The waveform representation may be used for the analysis, interpretive statements of the condition of the heart, and a graphical representation of the STJ level values. Reliability and/or accuracy of such waveform representation may be highly significant in a deciding a type of emergency medical treatment to be provided. Accordingly, processing unit 350 may also cause appropriate audible fonts to be emitted that indicate whether the waveform representation may be relied upon. In addition, the processing unit 350 may control delivery of other types of treatment therapy to the person via the defibrillation electrodes 372 and 374, such as cardioversion or pacing therapy.

The processing unit 350 may include one or more general-purpose processors, special purpose processors (*e.g.*, digital signal processors (DSP), application specific integrated circuits (ASIC), graphic processing units, etc.), or any other suitable processing unit or controller. The processing unit 350 can be configured to execute instructions (*e.g.*, computer-readable program instructions) that are stored in memory and may be executable to provide the functionality of the medical device described herein. For example, the processing unit 350 can execute instructions for causing the medical device to display an ECG waveform on the user interface 354 of the medical device 302.

The communication module 356 of the medical device 302 may be in communication with the processing unit 350. The communication module 356 may enable patient data, treatment information, CPR performance, system data, environmental data, etc. to be communicated between the medical device 302 and other devices, such as a remote assistance center and/or any other remote computing device. The communication module 356 may include one or more wireless or wireline interfaces that allow for both short-range communication and long range communication to one or more networks or to one or more remote devices. The wireless interfaces may provide for communication under one or more wireless communication protocols, such as Bluetooth, Wi-Fi (e.g., an institute of electrical and electronic engineers (IEEE) 802.xx protocol), Long-Term Evolution (LTE), cellular communications, near-field communication (NFC), radio-frequency identification (RFID), and/or other wireless communication protocols. Such wireline interfaces may include an Ethernet interface, USB interface, or similar interface to communicate via a wire, a twisted pair of wires, a coaxial cable, an optical link, a fiber-optic link, or other physical connection to a wireline network.

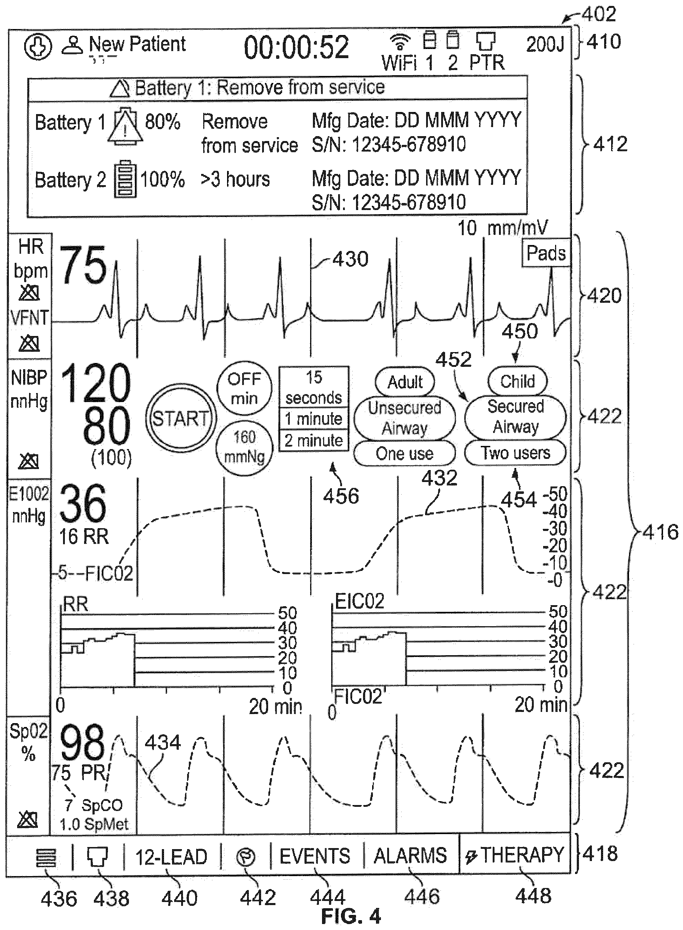

FIG. 4 illustrates a GUI 402, in accordance with an example implementation. The GUI 402 can be displayed by a medical device, such as the medical devices of Figures 1-3.

The GUI 402 may be configured to allow a user or rescuer to input information (e.g., parameters, variables, etc.) for enabling the medical device to select a cardiac or a resuscitation treatment (e.g., a CPR treatment) for a person experiencing a medical condition. For example, the GUI 402 may allow the user to input or select parameters related to the pitch, intensity and audio font of the SpO2 tones that represent sonifications of physiologic and device information, an age classification of a person 450 (e.g., adult or child/infant), an airway status 452 (e.g., whether the person has their airway secured by artificial airway), whether the CPR is being delivered by one or two persons 454, whether the, and/or other variables which may be used by the medical device to determine or select a CPR treatment protocol. The GUI 402 may display user-selectable user-interface items to allow a user to input or select time periods 456 for a CPR treatment

As shown in FIG. 4, the GUI 402 includes a status bar 410, a messaging area 412, a content area 416, and a navigation task bar 418. The messaging area 412 of the GUI 402 may display messages and information to the user or rescuer. The content area 416 of the GUI 402 may show patient data including physiologic parameters and waveforms output or displayed by the medical device as well as provided by physiologic sensors. The content area 416 can include multiple channels, including a primary channel 420 for displaying a primary waveform 430 and multiple secondary channels 422 for displaying secondary data, such as a secondary waveform 432. Although multiple secondary channels 422 are shown, in other examples, a GUI may only display a single secondary channel.

The GUI 402 may also be configured to display waveforms next to a side rectangle having a particular color and labelled by a physiologic parameter to which the waveform pertains. For example, the GUI includes the waveform 430 for heart rate (HR), the waveform 432 for End-tidal CO2 (EtCO2), which indicates the partial pressure or maximal concentration of carbon dioxide (CO2) at the end of an exhaled breath, and the waveform 434 for SpO2. The GUI can also display NIBP values for a patient or person being monitored and/or treated. Further, the GUI 402 may display a navigation task bar 418 at the bottom of the GUI 402.

The navigation task bar 418 may have various tabs and menu options. As shown, the navigation task bars 418 includes a menu button 436, a print button 438, a 12-Lead button 440, a generic event button 442, an events button 444, an alarms button 446, and therapy button 448.

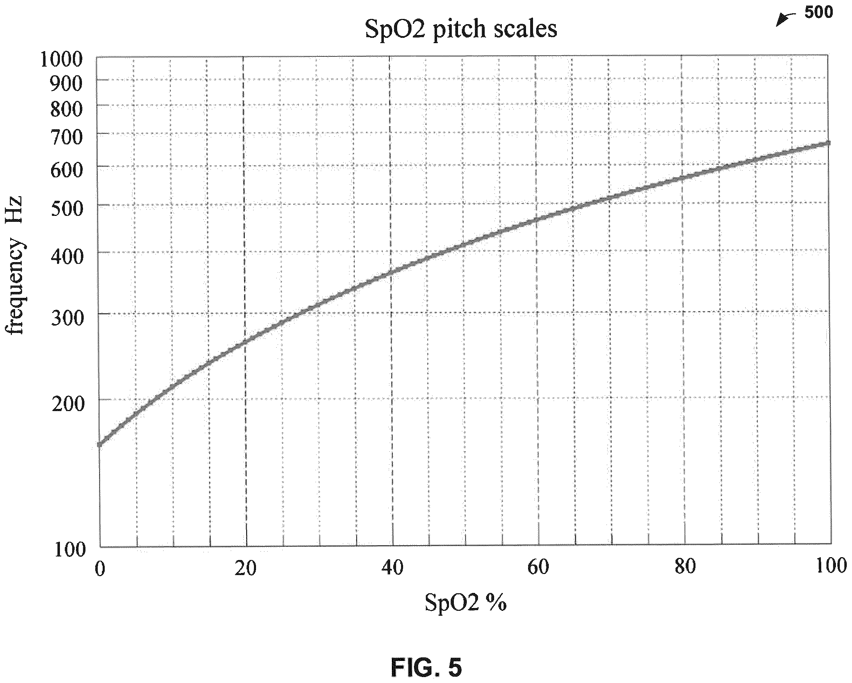

FIG. 5 is an example graph illustrating SpO2 values, in accordance with an example implementation. The graph 500 demonstrates how close in frequency SpO2 values from 90 to 100% sound. Barely 50 Hz separates 90 from 100%. Such 5 Hz increments are insufficient for reliable audible discrimination in this sub octave interval. As illustrated, a typical approach involves formulating a linear equation relating sound pitch to some displayed value. For example, pulse oximeters that provide pulse tones of variable pitch dependent on the measured oxygen saturation typically use a linear mapping between oxygen saturation and the sonic frequency of the pulse tone. In this scheme, the entire 100% to 0% range of possible oxygen saturation values is mapped across a range of approximately two musical octaves. However, human psychoacoustic perception of the relationship between the frequency of a sound and its musical pitch is logarithmic, rather than linear. Accordingly, referring again to the SpO2 example, certain ranges of SpO2 values may not have a clear frequency separation. For example, the linear mapping scheme can produce dramatically different behaviors at opposite ends of the range of possible oxygen saturation values. The change from 100% saturation to 90% saturation is generally mapped to a pitch change of less than a half tone, while the change from 30% saturation to 0% saturation is allocated nearly the entire lower octave, a more than 11-fold difference. Also, the emphasis is on a range that is both less clinically relevant and less accurately measured.

Pythagoras determined appropriate musical scales in the 6th century BC based on the logarithmic behavior of the Human Auditory System (HAS). Musical notes used in western music are constructed using a ratio of $\sqrt[12]{2}$ between notes. However, the 88 keys on a piano are not enough to represent the 100 notes required for SpO2 values. Also, for example, the 88 keys span a frequency range that exceeds a capacity for small speakers typically used in portable medical devices. For example, a typical 50 mm speaker has a frequency response of 350 to 5000 Hz between lower and upper resonance while the piano spans 29.135 to 4186 Hz for fundamentals of each key.

In some embodiments, the characteristic of the measurement data includes a magnitude of a value of the measurement data. The measurement data may be associated with a corresponding range of measurement values. Some embodiments involve mapping the range of measurement values in terms of intervals of musical notes. A typical approach by non-audio oriented engineers involves formulating a linear equation relating sound pitch to some displayed value. One popular SpO2 device utilizes the relationship below:

$$Tone\ Frequency = (SpO2\%) * 5 + 162\ Hz$$

(Eqn. 1)

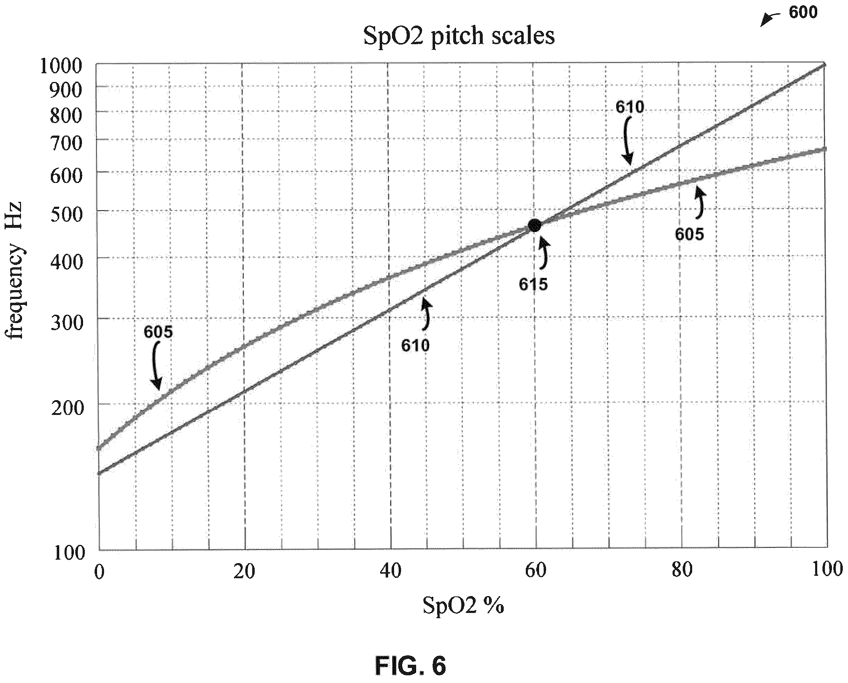

FIG. 6 is a comparison 600 of a scaled graph 605 and a linear graph 610 for SpO2 values, in accordance with an example implementation. In some embodiments, the device includes a pulse oximeter displaying SpO2 values in a range from 0-100%. While the linear mapping scheme of saturation to sonic frequency may be reasonable in some monitoring situations, it can be suboptimal, and even potentially hazardous, during certain emergency care procedures. For example, patients undergoing rapid sequence intubation (RSI) are at high risk for precipitous oxygen desaturation. An inability to promptly detect and correct such desaturation can result in significant patient harm including cardiac arrest. For an RSI patient with an initial oxygen saturation near 100%, a decrease in finger pulse oximeter oxygen saturation to 90% means that the true arterial saturation is already likely well below 90% and in a danger zone. In such circumstances, using a pitch change of less than a half tone to indicate the difference between "perfect" and "extreme danger" is unlikely to convey the seriousness of the situation, especially when a caregiver performing the intubation is cognitively distracted by the act of intubation.

In some embodiments, the intervals of musical notes correspond to a ratio of $\sqrt[36]{2}$ between successive notes. The scaled (logarithmic) graph 605 depicts a scale based on ratio of $\sqrt[36]{2}$ compared to the linear graph 610. The two graphs 605 and 610 intersect at point 615 that corresponds to a SpO2 value of 61%. Greater separation of frequency exists at 61% and above on the linear graph 610 while switching to the scaled graph 605 below 61% provides an advantage of moving the lowest frequencies required to a higher value. Below 38% audible detection is reliant upon second and higher harmonics introduced to the tones. Sine waves with a psycho-acoustically designed second, third, and fifth harmonic structure may be utilized for audibility in noisy environments. Square waves may generally waste energy on the sixth and higher harmonics, and are not useful for tone audibility.

As described herein, a compromise that will fit 100 notes into the limits of the speaker frequency response while enhancing pitch discrimination has been determined using a ratio of $\sqrt[36]{2}$ between notes. A linear scale provided by linear graph 610 may be used for the upper pitch starting point (*e.g.,* SpO2 100%) of 987.77 Hz above SpO2 of 60%, and a logarithmic scale provided by scaled graph 605 may be used at 60% and below. Such a hybrid approach can result in considerable improvement of auditory discrimination by creating a greater separation between the upper frequencies. Some embodiments may involve a first mapping of the SpO2 values, wherein the first mapping is a linear mapping in terms of intervals of musical notes for SpO2 values below 60%, and a second mapping of the SpO2 values, wherein the second mapping is a logarithmic mapping in terms of intervals of musical notes for SpO2 values above 60%. An example hybrid approach is summarized is Table 1 below:

**Table 1**

| SpO2 value *x* | Tone Base Pitch |
|---|---|
| *x* = 100% | 987.77 Hz |
| 99% ≥ *x* > 60% | frequency(x + 1) / 1.091441 Hz |
| 60% ≥ *x* ≥ 0% | *x* * 5 + 162 Hz |

In some examples, a hybrid approach may involve each 1% change being mapped to an even semi-tone or whole tone change over the range from 100% down to a first threshold, such as a value of *e.g.* 85%-90%, and the remainder of the range down to 0% being mapped with a different (*e.g.* linear) much more gradual mapping. This can help emphasize perceptibility of the initial drop from 100% towards and through the (first) clinically meaningful critical threshold, which may be a significant vital range and physiologic change for which prompt/continuous awareness is needed to maintain patient safety during an RSI.

In some embodiments, the characteristic of the measurement data may include a magnitude of a value of the measurement data, wherein the particular characteristic comprises a particular value, and wherein the one or more clinically meaningful thresholds comprises one or more threshold values.

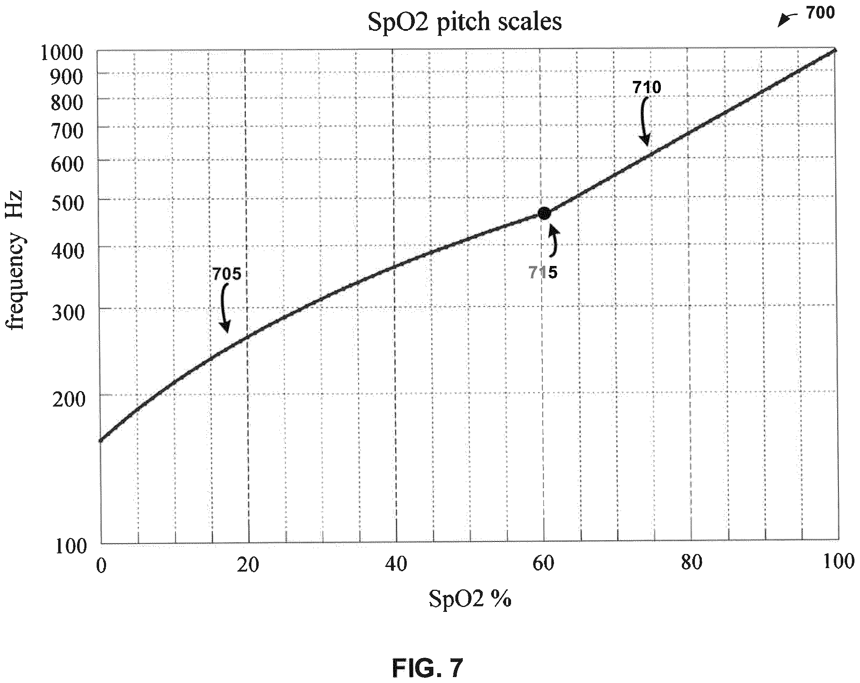

FIG. 7 illustrates an example hybrid pitch scale 700 for SpO2 values, in accordance with an example implementation. A hybrid approach can be adopted to fit 100 notes into the limits of the speaker frequency response while enhancing pitch discrimination. For example, the existing two octaves of potential pitches may be mapped to the 100% to 0% range of potential oxygen saturation values linearly in terms of musical interval, rather than linearly in terms of sonic frequency. This can facilitate greater perception of oxygen saturation changes at the higher end of the saturation range, where most patients are going to be operating, and where there is a greater need for situational awareness to prevent or rapidly respond to dangerous desaturation during critical procedures like RSI.

For example, the hybrid approach can involve a piecewise function that includes a first function 705 and a second function 710 that are connected at point 715. The point 715 corresponds to a SpO2 value of 60%. Accordingly, thirty-nine SpO2 values ranging from 100% to 61% (represented by the second function 710) may be fit into approximately one octave with improved auditory discrimination. The remaining sixty values below 61% (represented by the first function 705) may be fit into approximately 1.5 octaves and also present improved auditory discrimination. However this improvement may lack sufficient spread of tones for less musically inclined individuals.

Some embodiments involve identifying a plurality of intervals in the range from 0% - 100%, and associating a plurality of audible fonts with the corresponding plurality of identified intervals. The one or more clinically meaningful thresholds may include a transition of a SpO2 value from a given interval of the plurality of intervals to a next interval of the plurality of intervals. For example, the plurality of intervals may include a first interval of SpO2 values from 100% to 94%, a second interval of SpO2 values from 93% to 88%, and a third interval of SpO2 values from 87% to 0%. The plurality of audible fonts may include a first audio font corresponding to the first interval, a second audio font corresponding to the second interval, and a third audio font corresponding to the third interval, and wherein the one or more clinically meaningful thresholds comprises 94% or 88%.

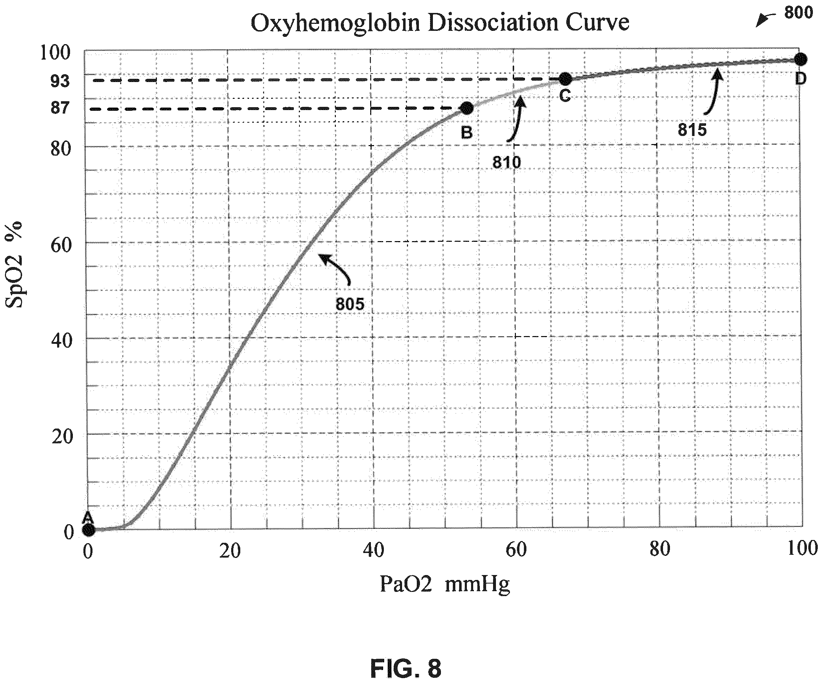

FIG. 8 illustrates an example dissociation curve 800 for multiple ranges of SpO2 values, in accordance with an example implementation. In some embodiments, in addition to pitch changes representing SpO2 values, modification of the tone timbre, which is referred to as "audible fonts," can increase the ability to audibly identify attributes of interest conveyed in the sonification. The oxyhemoglobin dissociation curve 800 illustrates multiple ranges of interest for SpO2 values. As illustrated, dissociation curve 800 may be a combination of three curves, a first curve 805 connecting point A (at an SpO2 value of 0%) to point B (at an SpO2 value of 87%), a second curve 810 connecting point B to point C (at an SpO2 value of 93%), and a third curve 815 connecting point C to point D (at an SpO2 value of 100%). For example, the first curve 805 represents a range of SpO2 values from 100% to 94%, the second curve 810 represents a range of SpO2 values from 93% to 88%, and the first curve 805 represents a range of SpO2 values below 87%.

Accordingly, a first audible font may be applied to the pitches for values represented by the first curve 805, a second audible font may be applied to pitches for values represented by the second curve 810, and a third audible font may be applied to pitches for values represented by the third curve 815. Such multiple audio fonts can greatly increase auditory identification even for non-musically inclined individuals.

In some embodiments, the particular audio font may be associated with a timbral characteristic.

FIG. 9A illustrates an example table 900A for a first set of pitch values for multiple ranges of SpO2 values, in accordance with an example implementation. Table 900A relates to the dissociation curve 800 of FIG. 8. In particular, table 900A illustrates example audible fonts. As illustrated by each of the columns of table 900A, the audible fonts may include a base frequency, a second harmonic, a third harmonic, and a fifth harmonic.

Portion 905 corresponds to the first curve 805 that represents a range of SpO2 values below 87%. Portion 905 of table 900A displays the SpO2 values from 52% to 87%. The remaining SpO2 values from 0% to 51% are displayed in table 900B of FIG. 9B. The first audible font or first timbral characteristic may be based on a noticeably very distorted or choppy timbre, indicating that the patient is in serious trouble, and/or that the measurement data is very unreliable. Accordingly, the first audible font may be based on a sine wave with a modulation depth of 30 Hz, and a modulation rate of 40 Hz.

Portion 910 corresponds to the second curve 810 that represents a range of SpO2 values from 93% to 88%. The second audible font or second timbral characteristic may be based on a noticeably less pure and/or choppier timbre, indicating that the patient/measurement is dipping into a range of concern. Accordingly, the second audible font may be based on a sine wave with a modulation depth of 15 Hz, and a modulation rate of 50 Hz.

Portion 915 corresponds to the third curve 815 representing a range of SpO2 values from 100% to 94%. The third audible font or third timbral characteristic may be based on a pure sign wave timbre indicating that the patient and/or measurement is within an acceptable range of SpO2 values. Accordingly, the third audible font may be based on a pure sine wave, with no modulation.

FIG. 9B illustrates an example table 900B for a second set of pitch values, in accordance with an example implementation. The remaining SpO2 values from 0% to 51% corresponding to portion 905 are shown. Table 900B is similar to table 900A in other aspects.

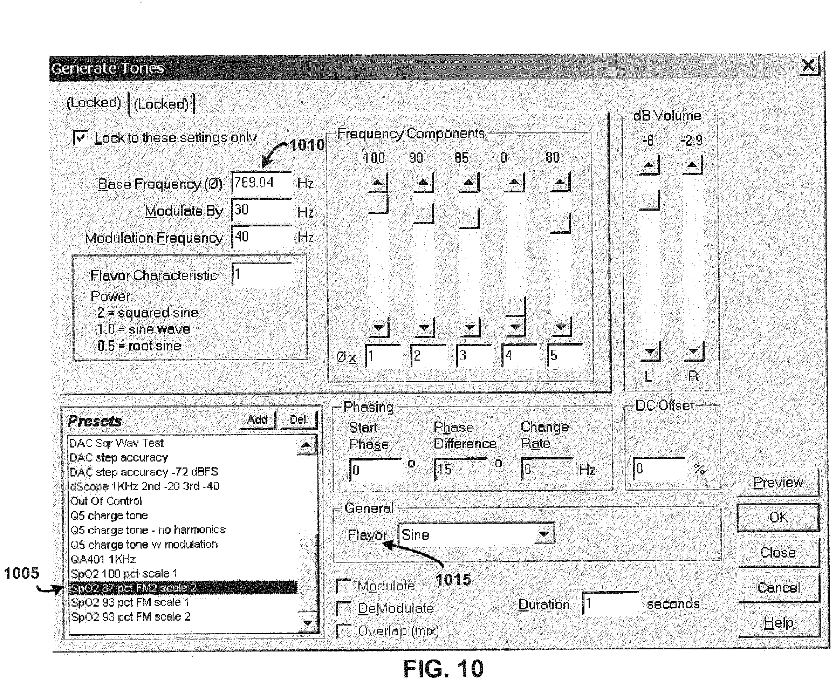

FIG. 10 illustrates an example interface to generate a modified sonification for a particular SpO2 value in a first range, in accordance with an example implementation. In some aspects, the example interface allows a user to select the first audible font or first timbral characteristic, "SpO2 87 pct FM2 scale 2" 1005. The first input values 1010 allow the user to enter the base frequency, an amount of modulation, and a modulation frequency. For example, the base frequency may be 769.04 Hz (corresponding to the base frequency for an SpO2 value of 87% as provided in portion 905 of table 900A of FIG. 9A), with a modulation depth of 30 Hz, and a modulation rate of 40 Hz.. Also, for example, first flavor 1015 may be selected to be a "sine" wave.

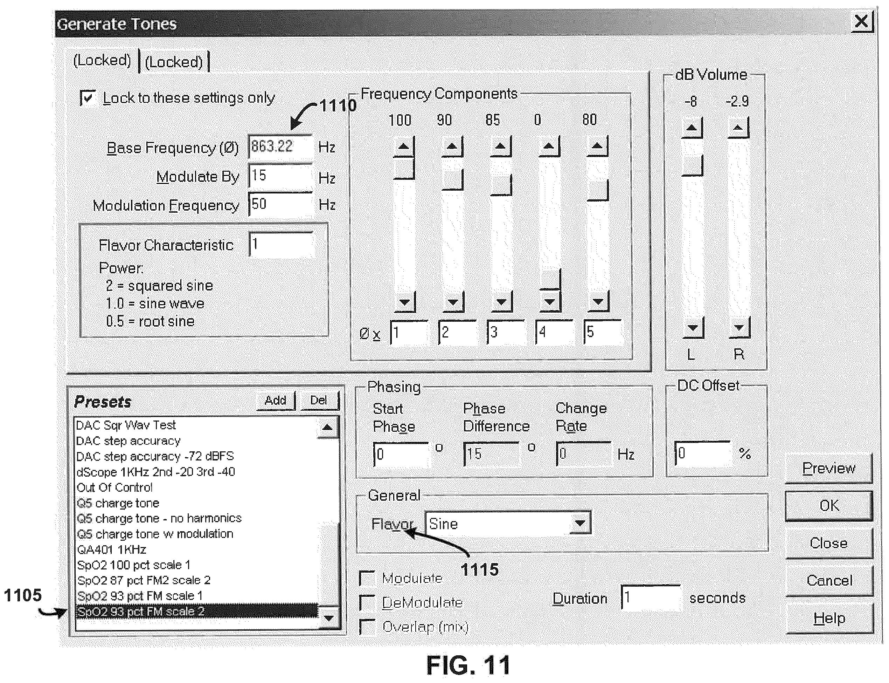

FIG. 11 illustrates an example interface to generate a modified sonification for a particular SpO2 value in a second range, in accordance with an example implementation. In some aspects, the example interface allows a user to select the second audible font or second timbral characteristic, "SpO2 93 pct FM2 scale 2" 1105. The second input values 1110 allow the user to enter the base frequency, an amount of modulation, and a modulation frequency. For example, the base frequency may be 863.22 Hz (corresponding to the base frequency for an SpO2 value of 93% as provided in portion 910 of table 900A of FIG. 9A), with a modulation depth of 15 Hz, and a modulation rate of 50 Hz. Also, for example, second flavor 1115 may be selected to be a "sine" wave.

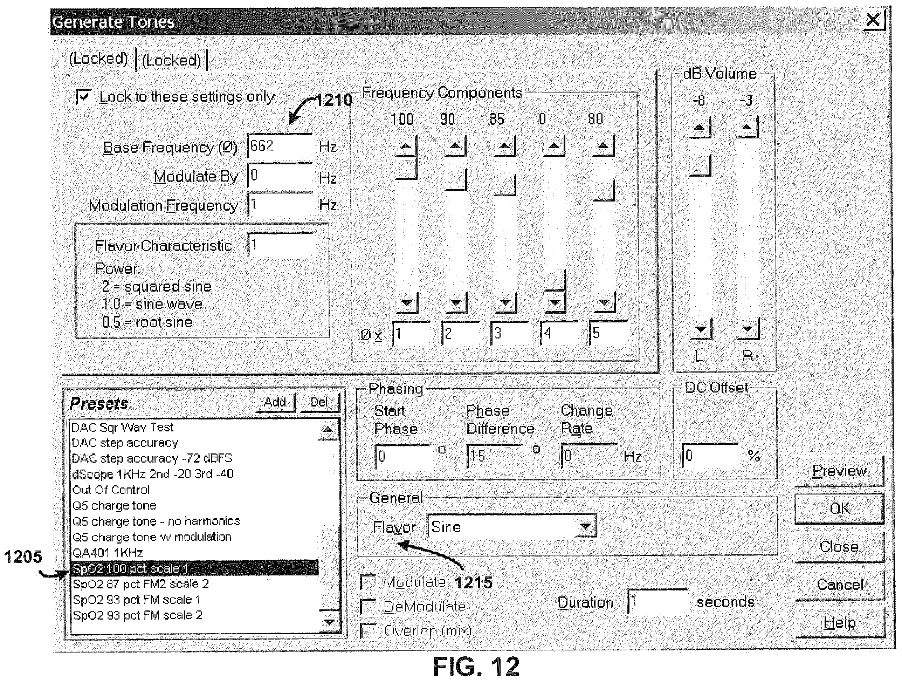

FIG. 12 illustrates an example interface to generate a modified sonification for a particular SpO2 value in a third range, in accordance with an example implementation. In some aspects, the example interface allows a user to select the third audible font or third timbral characteristic, "SpO2 100 pct scale 1" 1205. The third input values 1210 allow the user to enter the base frequency, an amount of modulation, and a modulation frequency. For example, the base frequency may be 662 Hz, and the modulation frequency may be 1 Hz, and no modulation may be applied. Also, for example, third flavor 1215 may be selected to be a "sine" wave.

In some embodiments, the tables 900A and 900B may be stored in memory. Also, for example, the input values and/or flavors may be pre-configured based on a preset audible font or timbral characteristic. For example, selection of "SpO2 87 pct FM2 scale 2" 1005 may automatically configure the first input values 1010, and/or the first flavor 1015. Likewise, a selection of "SpO2 93 pct FM2 scale 2" 1105 may automatically configure the second input values 1110, and/or the second flavor 1115, and a selection of "SpO2 100 pct scale 1" 1205 may automatically configure the third input values 1210, and/or the third flavor 1215.

In some scenarios, the medical device can be configured to apply different custom pitch mappings for different specific clinical applications. For example, the implementation described above can correspond a default mapping scheme that represents the highest saturation SpO2 values (e.g. the ~ 90-100% range) with three audio fonts. However, another example mapping scheme may involve changes for each fixed percentage drop (e.g., a 1% or 5% drop) between 100% and 90%. Such an implementation may be useful for Advanced Airway Management (AAM), where drops in oxygen saturation are anticipatable, fairly common, and can be extremely dangerous, and awareness of even the beginning of a drop from 100% to 99% to 98% can be of great value to a medical provider for purposes of maintaining patient safety.

For example, an AAM feature may involve a user identifying that an AAM procedure is to be followed. Subsequently, the user may activate a specific AAM support mode on the monitor/defibrillator, and as a part of the activated AAM Support Mode, the device can be configured to switch to using a different SpO2 pitch mapping scheme (instead of the default pitch mapping scheme with three audio fonts). Some implementations may involve combining the AAM feature with the "sound font" feature described previously. In some implementations, an activation of the AAM feature may cause the device to activate and/or modify the "sound font" feature, either related to the SpO2 parameter itself, or to potentially monitor another parameter.

In some embodiments, the device may include one or more measurement modes, wherein each measurement mode of the one or more measurement modes is associated with a corresponding range of measurement values. Such embodiments may involve mapping, for each measurement mode, the corresponding range of measurement values in terms of the intervals of musical notes, identifying, for each measurement mode, a plurality of intervals for the corresponding range of measurement values, and associating, for each measurement mode, a respective plurality of audible fonts with the corresponding plurality of intervals. Some example applications of the techniques described herein are provided for illustrative purposes.

In some embodiments, the one or more measurement modes may include a first measurement mode and a second measurement mode. Some embodiments involve receiving user indication to switch from the first measurement mode to the second measurement mode, and responsive to the user indication, switching from a first mapping of a first range of measurement values associated with the first measurement mode to a second mapping of a second range of measurement values associated with the second measurement mode. For example, the device may include a pulse oximeter displaying SpO2 values in a range from 0-100%. The first measurement mode may correspond to a default mode associated with a first plurality of intervals comprising a first interval of SpO2 values from 100% to 94%, a second interval of SpO2 values from 93% to 88%, and a third interval of SpO2 values from 87% to 0%. The second measurement mode may correspond to an Advanced Airway Management (AAM) mode associated with a second plurality of intervals comprising successive 1% drops from 100% in SpO2 values.

Some embodiments involve responsive to the switching from the first mapping to the second mapping, switching from a first plurality of audible fonts for the first mapping to a second plurality of audible fonts for the second mapping, and the playing of the audio signal may be based on the second plurality of audible fonts. For example, a SpO2 value for a patient may begin at 100%, then drop through certain thresholds. As described herein, a medical personnel may hear the pitch dropping, and may additionally hear different audio fonts (*e.g.*, changes to a timbre) as the values drop through these thresholds.

In another example embodiment, a blood pressure monitor may indicate that a blood pressure of a patient has crossed a clinically meaningful threshold. Existing NIBP measurement technology is typically a single (non-continuous) snapshot measurement/. The measurement may not be available until the time interval where pulse tones would exhibit an audio font signifying signal quality has passed. However, the techniques described herein may be applicable in the context of continuous blood pressure measurement, such as, for example, a continuous non-invasive blood pressure measurement (*e.g.*, obtained via an arterial cannula).

For example, in prehospital emergency care, a systolic pressure dropping below 90 mm Hg is a decision-making trigger threshold for a number of different emergency care scenarios. Accordingly, when the blood pressure monitor indicates that a systolic blood pressure has dipped below 90 mm Hg, a medical personnel may hear the pitch dropping, and may additionally hear different audio fonts (*e.g.,* changes to a timbre) as the values drop below 90 mm Hg. Also, for example, in the context of administering CPR, a diastolic pressure dropping below 30 mm Hg during CPR may trigger generation of an audio font.

In another example embodiment, a CPR metronome may use audio fonts to indicate whether the medical provider who is performing chest compressions is complying with the metronome rate guidance or not. In this embodiment, a medical device would be configured with an audible CPR metronome, and additionally with sensors and/or signal analysis algorithms to measure the rate of the compressions or ventilations being performed on the patient. The medical device may use different audio fonts for the metronome tones to indicate when the performed rate is close to the metronome guidance rate, vs when the performed rate diverges from the metronome rate guidance.

In another example embodiment, a medical personnel may hear different audio fonts applied to the SpO2 tones depending on the status of some other physiologic or treatment parameter that may influence the patient's arterial blood oxygen saturation. For example, if one or more aspects of the ventilation process is determined by the device to be suboptimal (such as a critically low ventilation rate, a significant airway leak, etc.), the audio font of the SpO2 tones may be changed to signify that the process of oxygenating the patient is compromised, and may thus be a cause of decreasing SpO2 values.

In another example embodiment, a medical personnel (*e.g.,* a caregiver) may have just walked into a room where the patient is. Regardless of the pitch they hear, a change in the audio font can indicate that a condition of the patient has changed. This can eliminate the need for the medical personnel to look at an actual measurement data displayed on the device, and/or rely on a pitch alone, thereby enabling swift action by the medical personnel. Such a reduction in response time can save the patient in life-threatening critical situations.

Some embodiments involve displaying, by the GUI, the particular characteristic of the measurement data. The audible change in the particular audio font may be generated synchronously with the displaying of the particular characteristic of the measurement data. For example, applying the techniques described herein, the NIBP measurement may be associated with a "sound font" where the "beep" can be a first sound (*e.g.,* a pleasant sound) indicating a high quality pressure signal, or a second sound (*e.g.,* a distorted sound) indicating a low quality pressure signal, based on a level of artifact in the underlying pressure signal that is being interpreted by the NIBP system to identify the pulses. This can provide a user immediate ongoing feedback about whether the NIBP measurement is proceeding "cleanly", or is being affected with artifact that will likely make the resulting values either incorrect or unobtainable. Since the NIBP measurement cycle can take several tens of seconds, this can add meaningful value to the user.

Signal quality of the waveform representing measurement data may be another issue that can be communicated. Especially in emergency care situations, SpO2 measurements calculated by a pulse oximeter may sometimes be erroneous, and many pulse oximeters can hold a stale value for longer than what may be considered safe. For example, a falsely reassuring high number may be displayed by the medical device. Caregivers have to review the displayed waveform to determine a quality of the waveform, before they can accept or reject the displayed value. A distinct audio font that indicates signal quality can remedy this situation, and provide a real-time audible notification of a quality of the displayed waveform. In some embodiments, the characteristic of the measurement data may include a quality of the measurement data, wherein the particular characteristic comprises a particular quality indicator, and wherein the one or more clinically meaningful thresholds comprises one or more threshold quality indicators.

In some embodiments, the particular characteristic may include a signal quality indicator, and the generating of the audible change in the particular audio font may involve generating of the audible change to reflect that the signal quality indicator has crossed a clinically meaningful quality threshold. For example, another application of the audio fonts can be to indicate signal quality for incoming patient data. For example, the NIBP feature is typically not configured to provide any information to a user about the signal quality of an underlying signal. However, a modified NIBP feature may involve a pulse tone for pulses detected by the NIBP system. For example, such pulse tones may be at the same pitch. Generally, the NIBP system does not measure SpO2, and there may be situations where a patient may not be linked to a SpO2 sensor. Accordingly, the pulse tone may involve adding a "beep" for each pulse that the NIBP system detects as a part of its NIBP measurement process.

In some embodiments, the clinically meaningful quality threshold may indicate a change from an unreliable signal to a reliable signal. In some embodiments, the clinically meaningful quality threshold may indicate a change from a reliable signal to an unreliable signal. For example, the measurement data may be faulty making the signal to become unreliable. For example, one or more sensors or electrodes attached to a patient may malfunction or become disconnected. Signal quality may also be based on a precision level of the recording of the signal, and a frequency of statistical sampling of the signal. Apart from physical factors, including device and/or sensor malfunctions, signal quality may be impacted by network coverage, bandwidth, distance from the source of the signal, interference from other signals, noise in transmission of the signal, and so forth. Accordingly, the clinically meaningful quality threshold may indicate a change from a reliable signal to an unreliable signal. Accordingly, an audio font may be played by the medical device to alert the user of the unreliable signal. When the signal is deemed to become reliable, a different audio font may be played by the medical device to alert the user of the reliable signal.

For example, pulse oximeters may take input raw data and perform mathematical operations to generate output measurement data and/or graphs that are provided to the medical personnel. Also, for example, in the event of a degraded input signal quality, a pulse oximeter may use a previous reading to generate a current reading. Such a process can produce false positives (*e.g.,* a SpO2 reading of 81% could be provided as 97%), as well as false negatives. Dependence on such erroneous values may jeopardize effective patient care.

Some embodiments involve training a machine learning model in a supervised manner based on training data comprising a plurality of waveforms, wherein each waveform of the plurality of waveforms is associated with a label indicating a quality of the waveform. Some embodiments involve training the machine learning model in an unsupervised manner by applying a classifier to training data comprising a plurality of waveforms, wherein the classifier outputs one or more classes indicative of a quality of an input waveform.

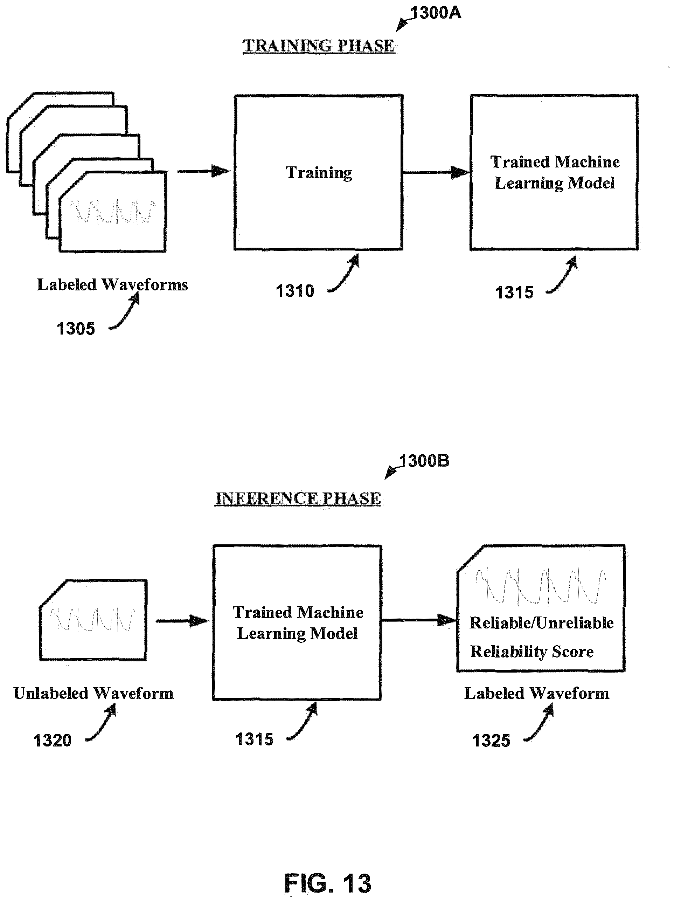

FIG. 13 illustrates training 1300A and inference 1300B phases for a machine learning model, in accordance with an example implementation. In the training phase 1300A, the training data may include a plurality of labeled waveforms 1305. Each waveform of the plurality of labeled waveforms 1305 may be associated with a label indicating a quality of the waveform (*e.g.*, reliable or unreliable). In some embodiments, each waveform of the plurality of labeled waveforms 1305 may be associated with a label indicating a reliability score (*e.g.*, reliable with a confidence level of 65%). Training 1310 of a machine learning model may be performed based on the plurality of labeled waveforms 1305 to output a trained machine learning model 1315. In some embodiments, the labeled data may be generated by subject matter experts, and the training may be supervised. In some embodiments, the training data may be synthetically generated based on a small sample of human annotated labeled data. For example, reliable and unreliable waveforms may be synthetically generated to train the machine learning model. Also, for example, noisy signals may be used to generate known distortions in waveforms, and such data may be used for training.

Also, for example, a feedback loop from the trained machine learning model may be used for reinforcement learning. For example, an activity by a user of the medical device may provide feedback as to the quality and/or reliability of the waveform. For example, indicating that a particular measurement data is unreliable may trigger additional actions by the user that may be detected by the medical device. The additional actions may include replacing one or more of the sensors, providing additional oxygen to the patient, performing CPR, generating an electrical pulse and delivering the electrical pulse to the patient, and so forth. A processor of the medical device may be configured to maintain a history of such causal relationships, and use such data in training (*e.g.*, via reinforcement learning techniques).

In some embodiments, machine learning models may be used to apply cluster analysis to waveforms. For example, similar waves may be clustered together based on various similarity measures (*e.g.*, cross-correlation of two waveforms). Also, for example, a computer vision model may be trained to automatically view and recognize waveforms as reliable or not.

The machine learning model 1315 may include, but not limited to artificial neural networks (*e.g.*, convolutional neural networks, recurrent neural networks, a Bayesian network, a hidden Markov model, a Markov decision process, a logistic regression function, a support vector machine, a statistical machine learning algorithm, and/or a heuristic machine learning system). Training 1310 may involve supervised, unsupervised, semi-supervised, and/or reinforcement learning techniques. In some embodiments, machine learning model 1315 may be a trained classifier such as a linear or non-linear classifier, a decision tree, a naive Bayes classifier, a *K*-nearest neighbors classifier, a support vector machine, or an artificial neural network.

In the inference phase 1300B, an unlabeled waveform 1320 may be input to the trained machine learning model 1315, and a labeled waveform 1325 may be output. In some embodiments, labeled waveform 1325 may be associated with an indication of a quality of the waveform. The indication of the quality may indicate whether the unlabeled waveform 1320 is reliable or unreliable. In some embodiments, labeled waveform 1325 may be associated with a reliability score. For example, the reliability score may provide a confidence level of the quality. For example, a "reliable" label with a confidence level of 30% may be deemed less reliable than a "reliable" label with a confidence level of 80%.

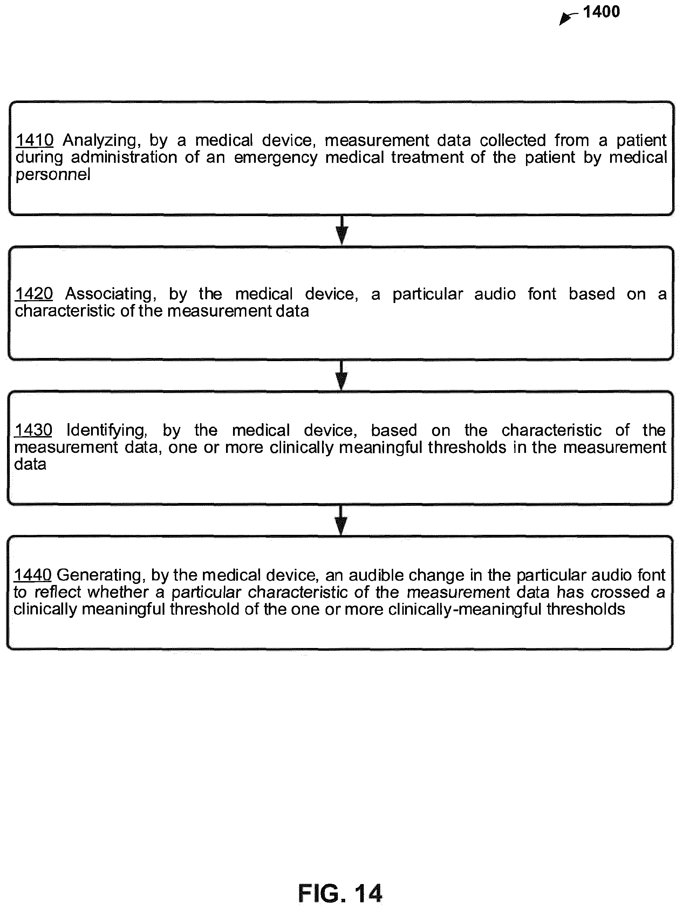

Figure 14 illustrates a flow chart of a method 1400 to provide a real-time audio feedback for use during administration of an emergency medical treatment of a patient by medical personnel, in accordance with example implementations. The method 1400 represents an example method that may include one or more operations, functions, or actions, as depicted by one or more of blocks 1410-1440, each of which may be performed by any of the medical devices or systems described herein. For instance, medical devices depicted in Figures 1-3 may enable execution of the method 1400.

Those skilled in the art will understand that the flowchart of Figure 14 herein illustrates functionality and operations of certain implementations of the present disclosure. In this regard, each block of the flowchart may represent a module, a segment, or a portion of program code, which includes one or more instructions executable by one or more processors for implementing specific logical functions or steps in the process. The program code may be stored on any type of computer readable medium, for example, such as a storage device including a disk or hard drive.

In addition, each block may represent circuitry that is wired to perform the specific logical functions in the process. Alternative implementations are included within the scope of the example implementations of the present application in which functions may be executed out of order from that shown or discussed, including substantially concurrent or in reverse order, depending on the functionality involved, as would be understood by those reasonably skilled in the art.

At block 1410, the method 1400 involves analyzing, by a medical device, measurement data collected from a patient during administration of an emergency medical treatment of the patient by the medical personnel. A medical device may be operated by a user to monitor and provide treatment or care to a person or patient experiencing a medical condition, such as cardiac arrest or any other cardiac rhythm abnormality. The medical device may be the medical device shown in Figures 1-3.

At block 1420, the method 1400 involves associating, by the medical device, a particular audio font based on a characteristic of the measurement data.

At block 1430, the method 1400 involves identifying, by the medical device, based on the characteristic of the measurement data, one or more clinically meaningful thresholds in the measurement data.

At block 1440, the method 1400 involves generating, by the medical device, an audible change in the particular audio font to reflect whether a particular characteristic of the measurement data has crossed a clinically meaningful threshold of the one or more clinically-meaningful thresholds.

The embodiments described herein can be realized in hardware, software, or a combination of hardware and software. For example, the embodiments can be realized in a centralized fashion in at least one computer system or in a distributed fashion where different elements are spread across interconnected computer systems. Any kind of computer system or other apparatus adapted for carrying out the methods described herein can be employed. Further, the embodiments described herein can be embedded in a computer program product, which includes all the features enabling the implementation of the operations described herein and which, when loaded in a computer system, can carry out these operations.

The flowcharts and block diagrams described herein illustrate the architecture, functionality, and operation of possible implementations of systems, methods, and apparatus in accordance with various illustrative embodiments. In this regard, each block in the flowcharts or block diagrams may represent a module, segment, or portion of code, which comprises one or more executable instructions for implementing the specified logical function or functions. It should also be noted that, in some alternative implementations, the functions noted in a block may occur out of the order noted in the drawings. For example, the functions of two blocks shown in succession may be executed substantially concurrently, or the functions of the blocks may sometimes be executed in the reverse order, depending upon the functionality involved.

Additionally, instances in this specification where one element is "coupled" to another element can include direct and indirect coupling. Direct coupling can be defined as one element coupled to and in some contact with another element. Indirect coupling can be defined as coupling between two elements not in direct contact with each other, but having one or more additional elements between the coupled elements. Further, as used herein, securing one element to another element can include direct securing and indirect securing. Additionally, as used herein, "adjacent" does not necessarily denote contact. For example, one element can be adjacent another element without being in contact with that element.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

By the term "substantially" and "about" used herein, it is meant that the recited characteristic, parameter, or value need not be achieved exactly, but that deviations or variations, including for example, tolerances, measurement error, measurement accuracy limitations and other factors known to skill in the art, may occur in amounts that do not preclude the effect the characteristic was intended to provide.

Unless otherwise indicated, the terms "first," "second," etc. are used herein merely as labels, and are not intended to impose ordinal, positional, or hierarchical requirements on the items to which these terms refer. Moreover, reference to, *e.g.,* a "second" item does not require or preclude the existence of, *e.g.,* a "first" or lower-numbered item, and/or, *e.g.,* a "third" or higher-numbered item.

While apparatus has been described with reference to certain examples, it will be understood by those skilled in the art that various changes can be made and equivalents can be substituted without departing from the scope of the claims. Therefore, it is intended that the present apparatus not be limited to the particular examples disclosed, but that the disclosed apparatus include all embodiments falling within the scope of the appended claims.

The present disclosure also provides for the following Examples that may be combined with features described herein above and vice versa.

Example 1. A medical device configured to provide a real-time audio feedback for use during administration of an emergency medical treatment of a patient by medical personnel, the device comprising:

> a memory; and
> a processor coupled to the memory, the processor configured to perform operations comprising:
>
>> analyzing measurement data collected from the patient;
>> associating a particular audio font based on a characteristic of the measurement data;
>> identifying, based on the characteristic of the measurement data, one or more clinically meaningful thresholds in the measurement data; and
>> generating an audible change in the particular audio font to reflect whether a particular characteristic of the measurement data has crossed a clinically meaningful threshold of the one or more clinically-meaningful thresholds.

Example 2. The device of Example 1, the operations further comprising:
playing, to an environment of the device, an audio signal indicating the audible change in the particular audio font.

Example 3. The device of Example 1, wherein the particular audio font is associated with a timbral characteristic.

Example 4. The device of Example 1, wherein the characteristic of the measurement data comprises a magnitude of a value of the measurement data, wherein the particular characteristic comprises a particular value, and wherein the one or more clinically meaningful thresholds comprises one or more threshold values.

Example 5. The device of Example 1, wherein the characteristic of the measurement data comprises a quality of the measurement data, wherein the particular characteristic comprises a particular quality indicator, and wherein the one or more clinically meaningful thresholds comprises one or more threshold quality indicators.

Example 6. The device of Example 1, further comprising a display screen configured to display a graphical user interface (GUI) comprising graphical elements representative of one or more aspects of the analyzed measurement data.

Example 7. The device of Example 6, the operations further comprising:

> displaying, by the GUI, the particular characteristic of the measurement data, and
> wherein the audible change in the particular audio font is generated synchronously with the displaying of the particular characteristic of the measurement data.

Example 8. The device of Example 1, wherein the characteristic of the measurement data comprises a magnitude of a value of the measurement data, and wherein the measurement data is associated with a corresponding range of measurement values, and the operations further comprising:
mapping the range of measurement values in terms of intervals of musical notes.

Example 9. The device of Example 8, wherein the intervals of musical notes correspond to a ratio of $\sqrt[36]{2}$ between successive notes.

Example 10. The device of Example 8, wherein the device comprises a pulse oximeter displaying SpO2 values in a range from 0-100%, and the operations further comprising:

a first mapping of the SpO2 values, wherein the first mapping is a linear mapping in terms of intervals of musical notes for SpO2 values below 60%; and
a second mapping of the SpO2 values, wherein the second mapping is a logarithmic mapping in terms of intervals of musical notes for SpO2 values above 60%.

Example 11. The device of Example 8, wherein the device comprises a pulse oximeter displaying SpO2 values in a range from 0-100%, and the operations further comprising:

identifying a plurality of intervals in the range from 0-100%; and
associating a plurality of audible fonts with the corresponding plurality of identified intervals, and
wherein the one or more clinically meaningful thresholds comprise a transition of a SpO2 value from a given interval of the plurality of intervals to a next interval of the plurality of intervals.

Example 12. The device of Example 11, wherein the plurality of intervals comprises a first interval of SpO2 values from 100% to 94%, a second interval of SpO2 values from 93% to 88%, and a third interval of SpO2 values from 87% to 0%, wherein the plurality of audible fonts comprises a first audio font corresponding to the first interval, a second audio font corresponding to the second interval, and a third audio font corresponding to the third interval, and wherein the one or more clinically meaningful thresholds comprises 94% or 88%.

Example 13. The device of Example 8, wherein the device comprises one or more measurement modes, wherein each measurement mode of the one or more measurement modes is associated with a corresponding range of measurement values, and the operations further comprising:

mapping, for each measurement mode, the corresponding range of measurement values in terms of the intervals of musical notes;
identifying, for each measurement mode, a plurality of intervals for the corresponding range of measurement values; and
associating, for each measurement mode, a respective plurality of audible fonts with the corresponding plurality of intervals.

Example 14. The device of Example 13, wherein the one or more measurement modes comprises a first measurement mode and a second measurement mode, and the operations further comprising:

receiving user indication to switch from the first measurement mode to the second measurement mode; and
responsive to the user indication, switching from a first mapping of a first range of measurement values associated with the first measurement mode to a second mapping of a second range of measurement values associated with the second measurement mode.

Example 15. The device of Example 14, the operations further comprising:

responsive to the switching from the first mapping to the second mapping, switching from a first plurality of audible fonts for the first mapping to a second plurality of audible fonts for the second mapping, and
wherein the playing of the audio signal is based on the second plurality of audible fonts.

Example 16. The device of Example 15, wherein the device comprises a pulse oximeter displaying SpO2 values in a range from 0-100%, wherein the first measurement mode corresponds to a default mode associated with a first plurality of intervals comprising a first interval of SpO2 values from 100% to 94%, a second interval of SpO2 values from 93% to 88%, and a third interval of SpO2 values from 87% to 0%, wherein the second measurement mode corresponds to an Advanced Airway Management (AAM) mode associated with a second plurality of intervals comprising successive 1% drops from 100% in SpO2 values.

Example 17. The device of Example 1, wherein the characteristic of the measurement data comprises a quality of the measurement data, wherein the particular characteristic comprises a signal quality indicator, and wherein the generating of the audible change in the particular audio font comprises generating of the audible change to reflect that the signal quality indicator has crossed a clinically meaningful quality threshold.

Example 18. The device of Example 17, wherein the clinically meaningful quality threshold indicates a change from an unreliable signal to a reliable signal.

Example 19. The device of Example 17, wherein the clinically meaningful quality threshold indicates a change from a reliable signal to an unreliable signal.

Example 20. The device of Example 17, the operations further comprising:
applying a trained machine learning model to determine the quality of the measurement data.

Example 21. The device of Example 17, the operations further comprising:
training the machine learning model in a supervised manner based on training data comprising a plurality of waveforms, wherein each waveform of the plurality of waveforms is associated with a label indicating a quality of the waveform.

Example 22. The device of Example 17, the operations further comprising:
training the machine learning model in an unsupervised manner by applying a classifier to training data comprising a plurality of waveforms, wherein the classifier outputs one or more classes indicative of a quality of an input waveform.

Example 23. The device of Example 1, wherein the device comprises a defibrillator or a patient monitoring device.

Example 24. A method to provide a real-time audio feedback for use during administration of an emergency medical treatment of a patient by medical personnel, comprising:

analyzing, by a medical device, measurement data collected from the patient;
associating, by the medical device, a particular audio font based on a characteristic of the measurement data;
identifying, by the medical device, based on the characteristic of the measurement data, one or more clinically meaningful thresholds in the measurement data; and
generating, by the medical device, an audible change in the particular audio font to reflect whether a particular characteristic of the measurement data has crossed a clinically meaningful threshold of the one or more clinically-meaningful thresholds.

Example 25. The method of Example 24, wherein the medical device comprises a defibrillator or a patient monitoring device.

Example 26. A non-transitory computer-readable medium configured to provide a real-time audio feedback for use during administration of an emergency medical treatment of a patient by medical personnel, comprising:

a memory; and
a processor coupled to the memory, the processor configured to perform operations comprising:

analyzing, by a medical device, measurement data collected from the patient;
associating, by the medical device, a particular audio font based on a characteristic of the measurement data;
identifying, by the medical device, based on the characteristic of the measurement data, one or more clinically meaningful thresholds in the measurement data; and
generating, by the medical device, an audible change in the particular audio font to reflect whether a particular characteristic of the measurement data has crossed a clinically meaningful threshold of the one or more clinically-meaningful thresholds.

Example 27. The non-transitory computer-readable medium of Example 26, wherein the medical device comprises a defibrillator or a patient monitoring device.

## Claims

1. A medical device configured to provide a real-time audio feedback for use during administration of an emergency medical treatment of a patient by medical personnel, the device comprising:

a memory; and

a processor coupled to the memory, the processor configured to perform operations comprising:

analyzing measurement data collected from the patient;
associating a particular audio font based on a characteristic of the measurement data;
identifying, based on the characteristic of the measurement data, one or more clinically meaningful thresholds in the measurement data; and
generating an audible change in the particular audio font to reflect whether a particular characteristic of the measurement data has crossed a clinically meaningful threshold of the one or more clinically-meaningful thresholds.

2. The device of claim 1, wherein the particular audio font is associated with a timbral characteristic and/or wherein the operations further comprise playing, to an environment of the device, an audio signal indicating the audible change in the particular audio font.

3. The device of claim 1 or 2, wherein the characteristic of the measurement data comprises:

(i) a magnitude of a value of the measurement data, wherein the particular characteristic comprises a particular value, and wherein the one or more clinically meaningful thresholds comprises one or more threshold values; and/or
(ii) a quality of the measurement data, wherein the particular characteristic comprises a particular quality indicator, and wherein the one or more clinically meaningful thresholds comprises one or more threshold quality indicators.

4. The device of any one of claims 1 to 3, further comprising a display screen configured to display a graphical user interface, GUI, comprising graphical elements representative of one or more aspects of the analyzed measurement data; wherein, optionally, the operations further comprise:

displaying, by the GUI, the particular characteristic of the measurement data, and
wherein the audible change in the particular audio font is generated synchronously with the displaying of the particular characteristic of the measurement data.

5. The device of any one of claims 1 to 4, wherein the characteristic of the measurement data comprises a magnitude of a value of the measurement data, and wherein the measurement data is associated with a corresponding range of measurement values, and the operations further comprising:
mapping the range of measurement values in terms of intervals of musical notes, wherein, optionally, the intervals of musical notes correspond to a ratio of $\sqrt[36]{2}$ between successive notes.

6. The device of claim 5, wherein the device comprises a pulse oximeter displaying SpO2 values in a range from 0-100%, the operations further comprising:

(i) - a first mapping of the SpO2 values, wherein the first mapping is a linear mapping in terms of intervals of musical notes for SpO2 values below 60%, and

- a second mapping of the SpO2 values, wherein the second mapping is a logarithmic mapping in terms of intervals of musical notes for SpO2 values above 60%; and/or

(ii) - identifying a plurality of intervals in the range from 0-100%, and

- associating a plurality of audible fonts with the corresponding plurality of identified intervals, wherein the one or more clinically meaningful thresholds comprise a transition of a SpO2 value from a given interval of the plurality of intervals to a next interval of the plurality of intervals; optionally wherein the plurality of intervals comprises a first interval of SpO2 values from 100% to 94%, a second interval of SpO2 values from 93% to 88%, and a third interval of SpO2 values from 87% to 0%, wherein the plurality of audible fonts comprises a first audio font corresponding to the first interval, a second audio font corresponding to the second interval, and a third audio font corresponding to the third interval, and wherein the one or more clinically meaningful thresholds comprises 94% or 88%.

7. The device of claim 5 or 6, wherein the device comprises one or more measurement modes, wherein each measurement mode of the one or more measurement modes is associated with a corresponding range of measurement values, and the operations further comprising:

   mapping, for each measurement mode, the corresponding range of measurement values in terms of the intervals of musical notes;
   identifying, for each measurement mode, a plurality of intervals for the corresponding range of measurement values; and
   associating, for each measurement mode, a respective plurality of audible fonts with the corresponding plurality of intervals.

8. The device of claim 7, wherein the one or more measurement modes comprises a first measurement mode and a second measurement mode, and the operations further comprising:

   receiving user indication to switch from the first measurement mode to the second measurement mode; and
   responsive to the user indication, switching from a first mapping of a first range of measurement values associated with the first measurement mode to a second mapping of a second range of measurement values associated with the second measurement mode;
   the operations optionally further comprising:
   responsive to the switching from the first mapping to the second mapping, switching from a first plurality of audible fonts for the first mapping to a second plurality of audible fonts for the second mapping, wherein the playing of the audio signal is based on the second plurality of audible fonts.

9. The device of claim 7 or 8, wherein the device comprises a pulse oximeter displaying SpO2 values in a range from 0-100%, wherein the first measurement mode corresponds to a default mode associated with a first plurality of intervals comprising a first interval of SpO2 values from 100% to 94%, a second interval of SpO2 values from 93% to 88%, and a third interval of SpO2 values from 87% to 0%, wherein the second measurement mode corresponds to an Advanced Airway Management, AAM, mode associated with a second plurality of intervals comprising successive 1% drops from 100% in SpO2 values.

10. The device of any one of claims 1 to 9, wherein the characteristic of the measurement data comprises a quality of the measurement data, wherein the particular characteristic comprises a signal quality indicator, and wherein the generating of the audible change in the particular audio font comprises generating of the audible change to reflect that the signal quality indicator has crossed a clinically meaningful quality threshold; optionally wherein the clinically meaningful quality threshold indicates a change from an unreliable signal to a reliable signal and/or a change from a reliable signal to an unreliable signal.

11. The device of claim 10, the operations further comprising:

   applying a trained machine learning model to determine the quality of the measurement data; and/or
   training the machine learning model in a supervised manner based on training data comprising a plurality of waveforms, wherein each waveform of the plurality of waveforms is associated with a label indicating a quality of the waveform; and/or
   training the machine learning model in an unsupervised manner by applying a classifier to training data comprising a plurality of waveforms, wherein the classifier outputs one or more classes indicative of a quality of an input waveform.

12. The device of any one of claims 1 to 11, wherein the device comprises a defibrillator or a patient monitoring device.

13. A method to provide a real-time audio feedback for use during administration of an emergency medical treatment of a patient by medical personnel, comprising:

   analyzing, by a medical device, measurement data collected from the patient;
   associating, by the medical device, a particular audio font based on a characteristic of the measurement data;
   identifying, by the medical device, based on the characteristic of the measurement data, one or more clinically meaningful thresholds in the measurement data; and
   generating, by the medical device, an audible change in the particular audio font to reflect whether a particular characteristic of the measurement data has crossed a clinically meaningful threshold of the one or more clinically-meaningful thresholds.

**14.** A computer program including instructions which, when executed by a processor, cause the processor to perform the method of claim 13.

**15.** A memory storing the computer program of claim 14.

FIG. 1

FIG. 2

EP 4 579 676 A1

**FIG. 3**

302 MEDICAL DEVICE

302

USER/
RESCUER

350
PROCESSING
UNIT

USER
INTER
-FACE

354

MEASUREMENT
MODULE

366

368

SENSOR
INTERFACE

SENSORS

386    384

DEFIBRILLATOR
ELECTRODES

358

394

POWER
SOURCE

392
BATTERY UNIT

390
BATTERY UNIT

362
ENERGY
STORAGE
MODULE

364
DIS-
CHARGE
CIRCUIT

376

378

370
DEFIB.
INTER
FACE

380

372

374

382

MEMORY
UNIT

COMMUNICATION
MODULE

CHARGING
MODULE

352          356          360

EP 4 579 676 A1

FIG. 4

SpO2 pitch scales

FIG. 5

EP 4 579 676 A1

FIG. 6

EP 4 579 676 A1

SpO2 pitch scales

FIG. 7

EP 4 579 676 A1

FIG. 8

EP 4 579 676 A1

900A

| SpO2 Value | Base (Fundamental) Frequency F1 (Hz) | 2nd Harmonic F2 (Hz) @ 90% Relative Amplitude | 3rd Harmonic F3 (Hz) @ 85% Relative Amplitude | 5th Harmonic F5 (Hz) @ 80% Relative Amplitude |
|---|---|---|---|---|
| 100 | 987.77 | 1975.54 | 2963.31 | 4938.85 |
| 99 | 968.93 | 1937.87 | 2906.80 | 4844.67 |
| 98 | 950.46 | 1900.91 | 2851.37 | 4752.28 |
| 97 | 932.33 | 1864.66 | 2796.99 | 4661.65 |
| 96 | 914.55 | 1829.10 | 2743.65 | 4572.76 |
| 95 | 897.11 | 1794.22 | 2691.33 | 4485.55 |
| 94 | 880.00 | 1760.01 | 2640.01 | 4400.02 |
| 93 | 863.22 | 1726.44 | 2589.66 | 4316.11 |
| 92 | 846.76 | 1693.52 | 2540.28 | 4233.80 |
| 91 | 830.61 | 1661.22 | 2491.84 | 4153.06 |
| 90 | 814.77 | 1629.55 | 2444.32 | 4073.86 |
| 89 | 799.23 | 1598.47 | 2397.70 | 3996.17 |
| 88 | 783.99 | 1567.99 | 2351.98 | 3919.97 |
| 87 | 769.04 | 1538.09 | 2307.13 | 3845.21 |
| 86 | 754.38 | 1508.75 | 2263.13 | 3771.89 |
| 85 | 739.99 | 1479.98 | 2219.97 | 3699.96 |
| 84 | 725.88 | 1451.76 | 2177.64 | 3629.40 |
| 83 | 712.04 | 1424.07 | 2136.11 | 3560.19 |
| 82 | 698.46 | 1396.92 | 2095.38 | 3492.29 |
| 81 | 685.14 | 1370.28 | 2055.42 | 3425.70 |
| 80 | 672.07 | 1344.15 | 2016.22 | 3360.37 |
| 79 | 659.26 | 1318.51 | 1977.77 | 3296.29 |
| 78 | 646.69 | 1293.37 | 1940.06 | 3233.43 |
| 77 | 634.35 | 1268.71 | 1903.06 | 3171.77 |
| 76 | 622.26 | 1244.51 | 1866.77 | 3111.28 |
| 75 | 610.39 | 1220.78 | 1831.17 | 3051.95 |
| 74 | 598.75 | 1197.50 | 1796.25 | 2993.75 |
| 73 | 587.33 | 1174.66 | 1761.99 | 2936.66 |
| 72 | 576.13 | 1152.26 | 1728.39 | 2880.66 |
| 71 | 565.14 | 1130.29 | 1695.43 | 2825.72 |
| 70 | 554.37 | 1108.73 | 1663.10 | 2771.84 |
| 69 | 543.80 | 1087.59 | 1631.39 | 2718.98 |
| 68 | 533.43 | 1066.85 | 1600.28 | 2667.13 |
| 67 | 523.25 | 1046.51 | 1569.76 | 2616.26 |
| 66 | 513.27 | 1026.55 | 1539.82 | 2566.37 |
| 65 | 503.49 | 1006.97 | 1510.46 | 2517.43 |
| 64 | 493.89 | 987.77 | 1481.66 | 2469.43 |
| 63 | 484.47 | 968.93 | 1453.40 | 2422.33 |
| 62 | 475.23 | 950.46 | 1425.68 | 2376.14 |
| 61 | 466.17 | 932.33 | 1398.50 | 2330.83 |
| 60 | 462.00 | 924.00 | 1386.00 | 2310.00 |
| 59 | 457.00 | 914.00 | 1371.00 | 2285.00 |
| 58 | 452.00 | 904.00 | 1356.00 | 2260.00 |
| 57 | 447.00 | 894.00 | 1341.00 | 2235.00 |
| 56 | 442.00 | 884.00 | 1326.00 | 2210.00 |
| 55 | 437.00 | 874.00 | 1311.00 | 2185.00 |
| 54 | 432.00 | 864.00 | 1296.00 | 2160.00 |
| 53 | 427.00 | 854.00 | 1281.00 | 2135.00 |
| 52 | 422.00 | 844.00 | 1266.00 | 2110.00 |

915

910

905

## FIG. 9A

900B

905

| SpO2 Value | Base (Fundamental) Frequency F1 (Hz) | 2nd Harmonic F2 (Hz) @ 90% Relative Amplitude | 3rd Harmonic F3 (Hz) @ 85% Relative Amplitude | 5th Harmonic F5 (Hz) @ 80% Relative Amplitude |
|---|---|---|---|---|
| 51 | 417.00 | 834.00 | 1251.00 | 2085.00 |
| 50 | 412.00 | 824.00 | 1236.00 | 2060.00 |
| 49 | 407.00 | 814.00 | 1221.00 | 2035.00 |
| 48 | 402.00 | 804.00 | 1206.00 | 2010.00 |
| 47 | 397.00 | 794.00 | 1191.00 | 1985.00 |
| 46 | 392.00 | 784.00 | 1176.00 | 1960.00 |
| 45 | 387.00 | 774.00 | 1161.00 | 1935.00 |
| 44 | 382.00 | 764.00 | 1146.00 | 1910.00 |
| 43 | 377.00 | 754.00 | 1131.00 | 1885.00 |
| 42 | 372.00 | 744.00 | 1116.00 | 1860.00 |
| 41 | 367.00 | 734.00 | 1101.00 | 1835.00 |
| 40 | 362.00 | 724.00 | 1086.00 | 1810.00 |
| 39 | 357.00 | 714.00 | 1071.00 | 1785.00 |
| 38 | 352.00 | 704.00 | 1056.00 | 1760.00 |
| 37 | 347.00 | 694.00 | 1041.00 | 1735.00 |
| 36 | 342.00 | 684.00 | 1026.00 | 1710.00 |
| 35 | 337.00 | 674.00 | 1011.00 | 1685.00 |
| 34 | 332.00 | 664.00 | 996.00 | 1660.00 |
| 33 | 327.00 | 654.00 | 981.00 | 1635.00 |
| 32 | 322.00 | 644.00 | 966.00 | 1610.00 |
| 31 | 317.00 | 634.00 | 951.00 | 1585.00 |
| 30 | 312.00 | 624.00 | 936.00 | 1560.00 |
| 29 | 307.00 | 614.00 | 921.00 | 1535.00 |
| 28 | 302.00 | 604.00 | 906.00 | 1510.00 |
| 27 | 297.00 | 594.00 | 891.00 | 1485.00 |
| 26 | 292.00 | 584.00 | 876.00 | 1460.00 |
| 25 | 287.00 | 574.00 | 861.00 | 1435.00 |
| 24 | 282.00 | 564.00 | 846.00 | 1410.00 |
| 23 | 277.00 | 554.00 | 831.00 | 1385.00 |
| 22 | 272.00 | 544.00 | 816.00 | 1360.00 |
| 21 | 267.00 | 534.00 | 801.00 | 1335.00 |
| 20 | 262.00 | 524.00 | 786.00 | 1310.00 |
| 19 | 257.00 | 514.00 | 771.00 | 1285.00 |
| 18 | 252.00 | 504.00 | 756.00 | 1260.00 |
| 17 | 247.00 | 494.00 | 741.00 | 1235.00 |
| 16 | 242.00 | 484.00 | 726.00 | 1210.00 |
| 15 | 237.00 | 474.00 | 711.00 | 1185.00 |
| 14 | 232.00 | 464.00 | 696.00 | 1160.00 |
| 13 | 227.00 | 454.00 | 681.00 | 1135.00 |
| 12 | 222.00 | 444.00 | 666.00 | 1110.00 |
| 11 | 217.00 | 434.00 | 651.00 | 1085.00 |
| 10 | 212.00 | 424.00 | 636.00 | 1060.00 |
| 9 | 207.00 | 414.00 | 621.00 | 1035.00 |
| 8 | 202.00 | 404.00 | 606.00 | 1010.00 |
| 7 | 197.00 | 394.00 | 591.00 | 985.00 |
| 6 | 192.00 | 384.00 | 576.00 | 960.00 |
| 5 | 187.00 | 374.00 | 561.00 | 935.00 |
| 4 | 182.00 | 364.00 | 546.00 | 910.00 |
| 3 | 177.00 | 354.00 | 531.00 | 885.00 |
| 2 | 172.00 | 344.00 | 516.00 | 860.00 |
| 1 | 167.00 | 334.00 | 501.00 | 835.00 |
| 0 | 162.00 | 324.00 | 486.00 | 810.00 |

## FIG. 9B

EP 4 579 676 A1

**Generate Tones**

(Locked) | (Locked) |

☑ Lock to these settings only          1010

Base Frequency (Ø)  769.04  Hz

Modulate By  30  Hz

Modulation Frequency  40  Hz

Flavor Characteristic  1
Power:
  2 = squared sine
  1.0 = sine wave
  0.5 = root sine

**Frequency Components**

100    90    85    0    80

Øx  1    2    3    4    5

**dB Volume**

-8    -2.9

L    R

**Presets**          Add | Del

DAC Sqr Wav Test
DAC step accuracy
DAC step accuracy -72 dBFS
dScope 1KHz 2nd -20 3rd -40
Out Of Control
Q5 charge tone
Q5 charge tone - no harmonics
Q5 charge tone w modulation
QA401 1KHz
SpO2 100 pct scale 1
SpO2 87 pct FM2 scale 2
SpO2 93 pct FM scale 1
SpO2 93 pct FM scale 2

1005

**Phasing**

Start Phase  |0  °
Phase Difference  15  °
Change Rate  |0  Hz

**DC Offset**

|0  %

**General**

Flavor  Sine          1015

☐ Modulate
☐ DeModulate        Duration  1  seconds
☐ Overlap (mix)

Preview

OK

Close

Cancel

Help

**FIG. 10**

EP 4 579 676 A1

**Generate Tones** ☒

(Locked) (Locked)

☑ Lock to these settings only

Base Frequency (∅) 863.22 Hz ← 1110

Modulate By 15 Hz

Modulation Frequency 50 Hz

Flavor Characteristic 1

Power:
 2 = squared sine
 1.0 = sine wave
 0.5 = root sine

Frequency Components

100 90 85 0 80

∅ x 1 2 3 4 5

dB Volume
-8 -2.9

L R

**Presets** Add Del

DAC Sqr Wav Test
DAC step accuracy
DAC step accuracy -72 dBFS
dScope 1KHz 2nd -20 3rd -40
Out Of Control
Q5 charge tone
Q5 charge tone - no harmonics
Q5 charge tone w modulation
QA401 1KHz
SpO2 100 pct scale 1
SpO2 87 pct FM2 scale 2
SpO2 93 pct FM scale 1
1105 → SpO2 93 pct FM scale 2

Phasing
Start Phase 0 °
Phase Difference 15 °
Change Rate 0 Hz

DC Offset
0 %

General
Flavor Sine ← 1115

☐ Modulate
☐ DeModulate
☐ Overlap (mix)

Duration 1 seconds

Preview

OK

Close

Cancel

Help

**FIG. 11**

FIG. 12

1300A

TRAINING PHASE

Labeled Waveforms

1305

Training

1310

Trained Machine
Learning Model

1315

1300B

INFERENCE PHASE

Unlabeled Waveform

1320

Trained Machine
Learning Model

1315

Reliable/Unreliable

Reliability Score

Labeled Waveform

1325

FIG. 13

1400

1410 Analyzing, by a medical device, measurement data collected from a patient during administration of an emergency medical treatment of the patient by medical personnel

1420 Associating, by the medical device, a particular audio font based on a characteristic of the measurement data

1430 Identifying, by the medical device, based on the characteristic of the measurement data, one or more clinically meaningful thresholds in the measurement data

1440 Generating, by the medical device, an audible change in the particular audio font to reflect whether a particular characteristic of the measurement data has crossed a clinically meaningful threshold of the one or more clinically-meaningful thresholds

FIG. 14

Europäisches
Patentamt

European
Patent Office

Office européen
des brevets

# EUROPEAN SEARCH REPORT

**Application Number**

EP 24 21 9474

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/224434 A1 (SILVER ANNEMARIE E [US] ET AL) 25 July 2019 (2019-07-25) <br> * paragraphs 7, 168, 5, 14, 20-22, 41-48, 136, 11, 222, 8, 114, 123 * <br> - - - - - | 1-15 | INV. <br> G16H20/40 <br> G16H40/63 <br> G16H50/20 <br> G16H50/70 |

**TECHNICAL FIELDS SEARCHED (IPC)**

G16H

The present search report has been drawn up for all claims

| Place of search | Date of completion of the search | Examiner |
|---|---|---|
| Munich | 5 May 2025 | Wittke, Claudia |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 21 9474

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2025

| Patent document cited in search report | Publication date | Patent family member(s) | Publication date |
|---|---|---|---|
| US 2019224434 A1 | 25-07-2019 | CN 111615412 A | 01-09-2020 |
| | | CN 118304526 A | 09-07-2024 |
| | | EP 3740269 A2 | 25-11-2020 |
| | | EP 4390967 A2 | 26-06-2024 |
| | | US 2019224434 A1 | 25-07-2019 |
| | | US 2022105291 A1 | 07-04-2022 |
| | | US 2024293634 A1 | 05-09-2024 |
| | | WO 2019143856 A2 | 25-07-2019 |

EPO FORM P0459

For more details about this annex : see Official Journal of the European Patent Office, No. 12/82